# EUROPEAN PATENT SPECIFICATION

(11) **EP 1 927 372 B1**
(45) Date of publication and mention of the grant of the patent: **03.06.2009**
(21) Application number: 06256102.2
(22) Date of filing: 29.11.2006
(51) Int. Cl.: A61M 5/32

(54) **Disposable syringe with built-in carpule**
Einwegspritze mit eingebauter Karpulle
Seringue jetable avec carpule intégrée

(43) Date of publication of application: 04.06.2008
(73) Proprietor: Shue, Ming-Jeng, Hsi Dist, Taichung City (TW); Shue, Philip, Hsi District Taichung City (TW); Huang, Deborah, Taichung City (TW)
(72) Inventor: Shue, Ming-Jeng, Hsi Dist, Taichung City (TW); Shue, Philip, Hsi District Taichung City (TW); Huang, Deborah, Taichung City (TW)
(74) Representative: Oxley, Robin John George

(56) References cited:
- WO-A-01/07104
- WO-A-98/13077
- WO-A-99/30759
- US-B1- 6 221 055

## Description

This invention relates to a disposable syringe, more particularly to a disposable syringe with a carpule.

In U.S. Patent No. 6, 221, 055 B1 for a retractable dental syringe as shown in Figs. 1 and 2, there is disclosed a pre-filled syringe 9, which includes a barrel 91 having an open widened rear end 911 to receive an operating end 973 of a plunger 97, a needle holder 93 for holding a double-ended needle 94, a push ring 92 for holding the needle holder 93 in the barrel 91, a biasing member 95 disposed between a front end 912 of the barrel 91 and the needle holder 93, and a carpule 96 positioned in the barrel 91 from the rear end 911. The carpule 96 has a front seal 963 disposed to seal a front end 962 thereof, and a slidable piston seal including an outer rim member 99 and a releasable core member 98 to seal a rear end 961 of the carpule 96 so as to define a sealed medicament chamber 964. The plunger 97 has inner and outer walls 971,972 parallel to and spaced apart from each other and connected at the operating end 973.

In operation, the rear end 961 of the carpule 96 is associated with the front of the plunger 97. Then the plunger and carpule assembly is introduced into the barrel 91 from the rear end 911, and the carpule 96 is moved forwardly until the front seal 963 is punctured by a communicating end 941 of the needle 94, as shown in Fig. 1. As a tip end 942 of the needle 94 is inserted into a patient, the plunger 97 is pressed forwardly to inject the contents of the chamber 964 through the needle 94, as shown in Fig. 2. At the end of an injection, the plunger 97 is further pressed to move the carpule 96 forwardly to remove the push ring 92, thereby freeing the needle holder 93 for retraction of the needle holder 93, the needle 94, the front seal 963 and the core member 98 into the carpule 96 by virtue of a biasing action of the biasing member 95, as shown in Fig. 3.

Although the syringe 9 can be operated to retract the needle 94 for safe disposal, the following drawbacks arise:
1. The assembly of the carpule 96 and the plunger 97 to the barrel 91 before the injection operation is complicated and inconvenient to conduct. In addition, with such construction, the carpule 96 cannot be accommodated in the barrel 91 in advance, and the carpule 96 and the barrel 91 have to be packed separately for subsequent assembly in use, thereby resulting in increased operation time.
2. Since the used needle 94 and the needle holder 93 are retracted within the inner wall 971 of the plunger 97, before triggering the biasing action of the biasing member 95, the respective retaining engagements between the push ring 92 and the needle holder 93, between the front seal 963 and the carpule 96, and between the core member 98 and the outer rim member 99 have to be released at the same time. Thus, the retraction of the used needle 94 may fail. Such complicated structural relationship makes the manufacture of the syringe 9 troublesome.

The object of the present invention is to provide a disposable syringe with a built-in carpule which ensures retraction of a used needle into a syringe barrel for safe disposal, which has a carpule that can be pre-assembled in the barrel before injection so that the injection operation is simplified and convenient to conduct, and which can be conveniently rendered unreusable after inject ion in a single operation.

According to this invention, the disposable syringe with a built-in carpule includes a barrel including front and rear barrel ends opposite to each other along an axis in an axial direction, a surrounding barrel wall which interconnects the front and rear barrel ends, and which includes front and rear wall portions that are disposed proximate to the front and rear barrel ends, respectively, and that define front and rear compartments, respectively. A needle cannula has a tip end which is disposed forwardly of the front barrel end in a position of use, and a communicating end which is opposite to the tip end along the axis. A seat member has a surrounding gripped portion surrounding the axis. A grip member is detachably retained on the rear wall portion, and is configured to be frictionally engaged with the gripped portion to provide a resisting force that guards the gripped portion against movement relative to the rear wall portion in ,the axial direction during a piercing action of the needle cannula for injection, and that permits disengagement of the grip member from the gripped portion so as to enable a subsequent movement of the seat member when the grip member is subj ected to a pressing force. A carpule has a tubular wall which is configured to be fit in the rear compartment, and which extends towards the grip member to terminate at a tubular edge surface, and front and rear seal members which are spaced apart from each other in the axial direction, and which, in cooperation with the tubular wall, form a sealed chamber for holding liquid medicament. The rear seal member is disposed to be movable to abut against the front seal member in an initial course so as to expel the liquidmedicament out of the sealed chamber, thereby placing the carpule in a used-up position. The front seal member has a mating surface which is configured to permit the communicating end to be retained with and to pass through the mating surface such that the needle cannula is in fluid communication with the sealed chamber, and such that once the rear seal member abuts against the front seal member in the used-up position, the pressing force is transmitted through the tubular wall to force the tubular edge surface against the grip member in a subsequent course so as to effect disengagement of the grip member from the gripped portion, thereby placing the seat member in a disengaging position, where the mating surface is closer to the front barrel end than when the carpule is in the used-up position. A plunger has a push end which is disposed to move the rear seal member forwardly along the tubular wall to bring the carpule to the used-up position, and to move the mating surface forwardly from the used-up position so as to permit the seat member to be placed in the disengaging position, and an operating end which is opposite to the push end in the axial direction, and which is disposed to extend outwardly of the rear barrel end to be subjected to an external force which is greater in the subsequent course than in the initial course. A biasing member is disposed in the front compartment such that, once the seat member is in the disengaging position, the biasing member biases the seat member together with the carpule towards the rear barrel end to retract the tip end of the needle cannula into the barrel, thereby placing the needle cannula in a disposal position.

Other features and advantages of the present invention will become apparent in the following detailed description of the preferred embodiments of the invention, with reference to the accompanying drawings, in which:

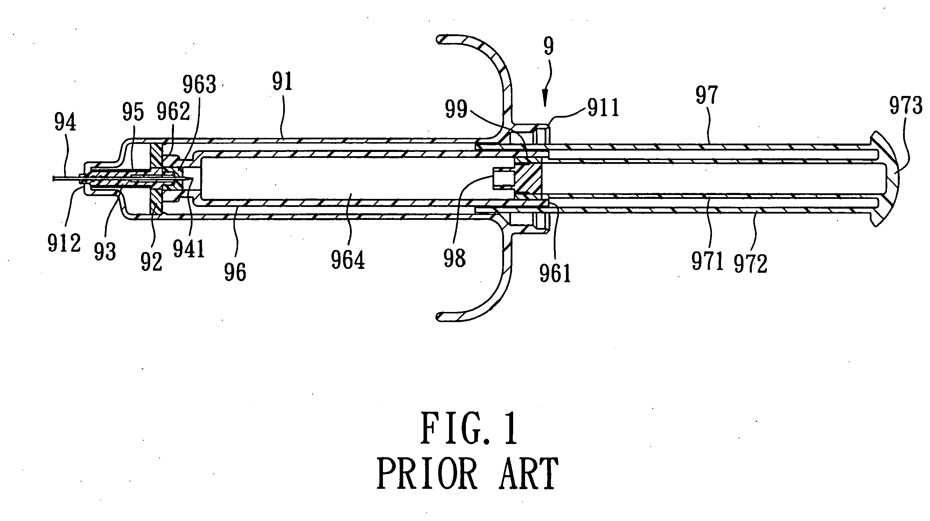

Fig. 1 is a sectional view of a conventional retractable syringe in a ready-to-use position;

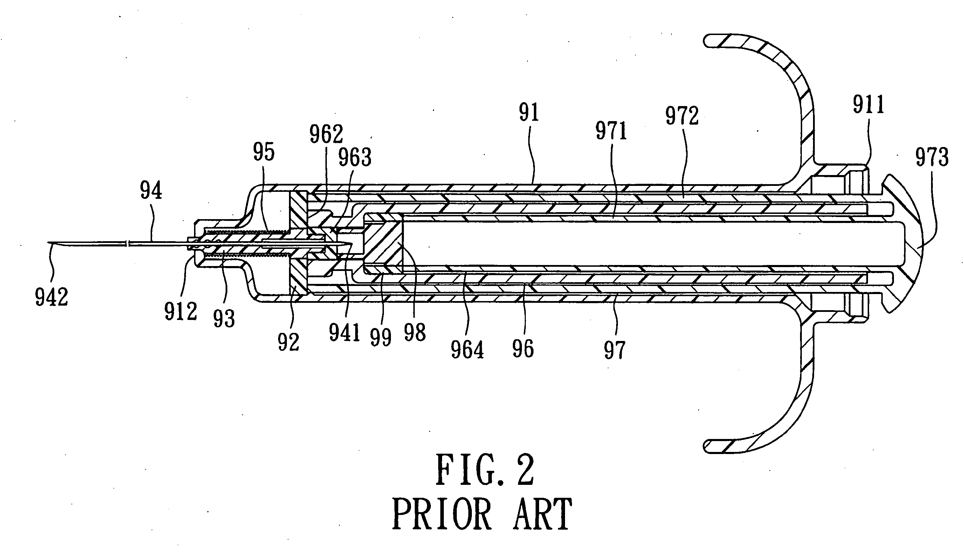

Fig. 2 is a sectional view of the conventional retractable syringe in a state after completion of an injection stroke;

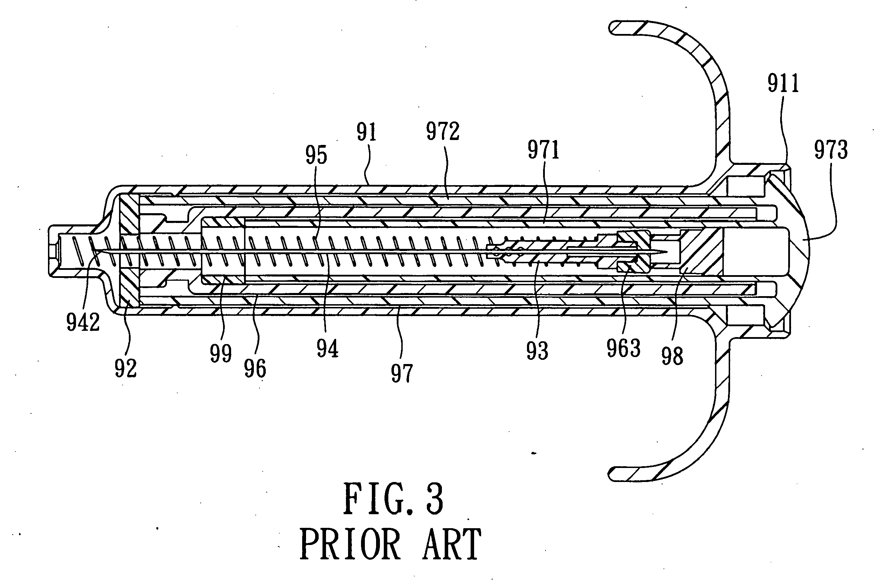

Fig. 3 is a sectional view of the conventional retractable syringe, showing that a needle-holding structure is fully retracted into a carpule;

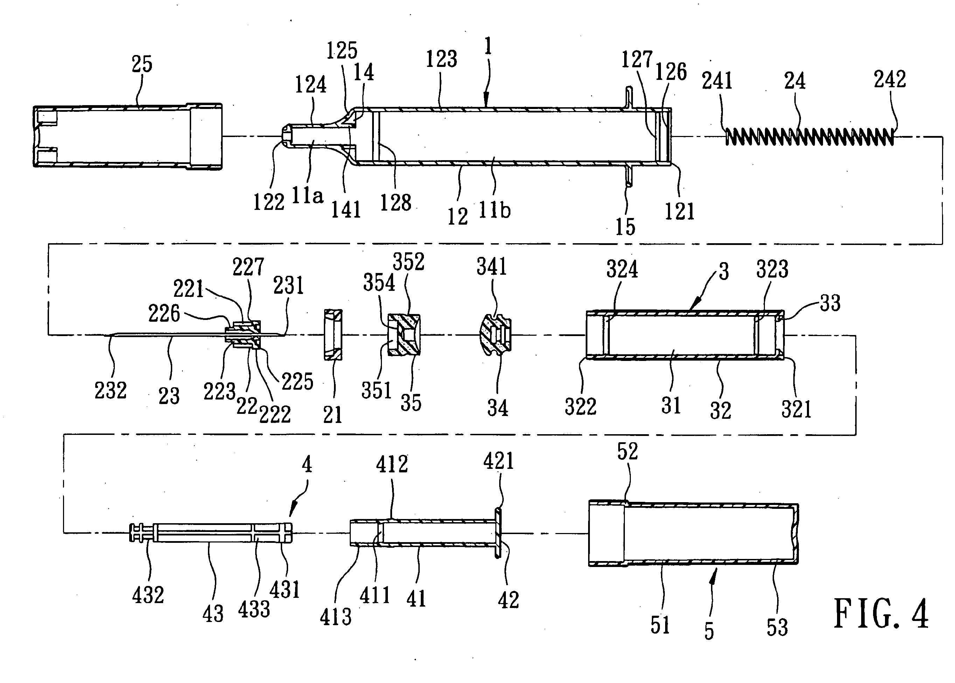

Fig. 4 is an exploded sectional view of the first preferred embodiment of a disposable syringe according to this invention;

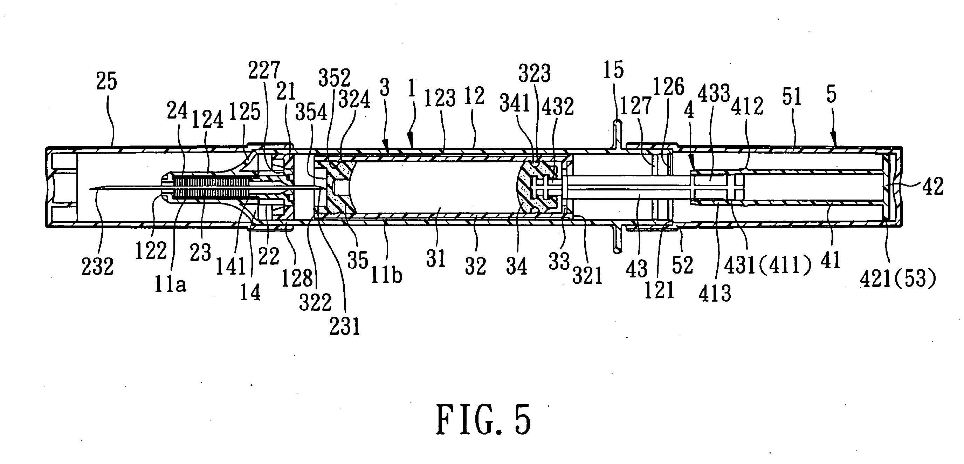

Fig. 5 is a sectional view of the first preferred embodiment in an initial assembled position;

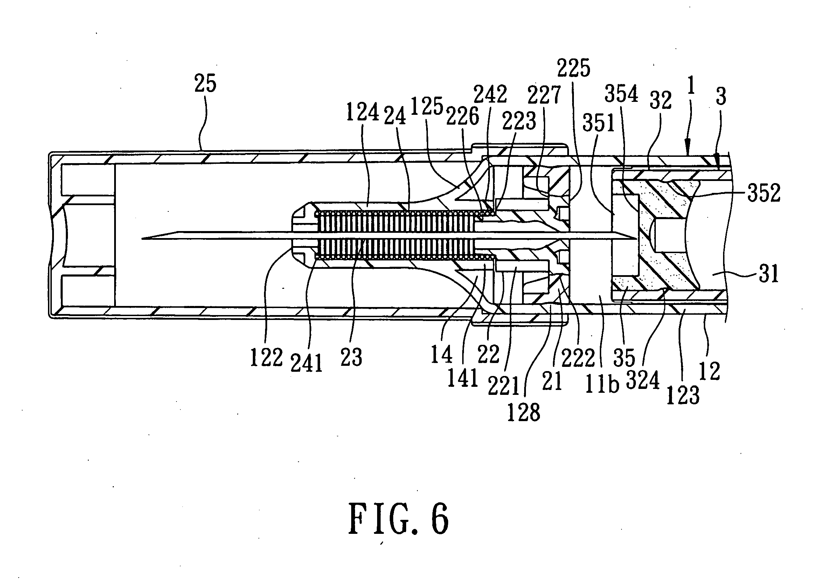

Fig. 6 is a fragmentary sectional view of the first preferred embodiment;

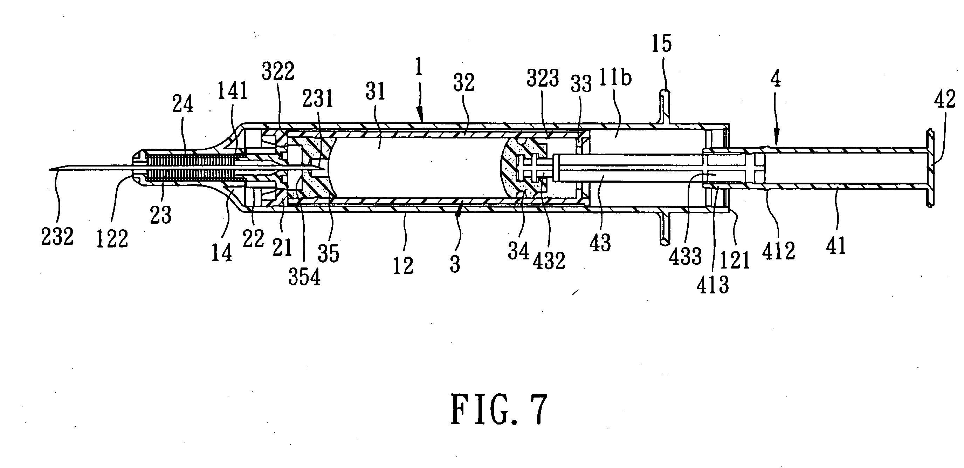

Fig. 7 is a sectional view of the first preferred embodiment during an injection stroke;

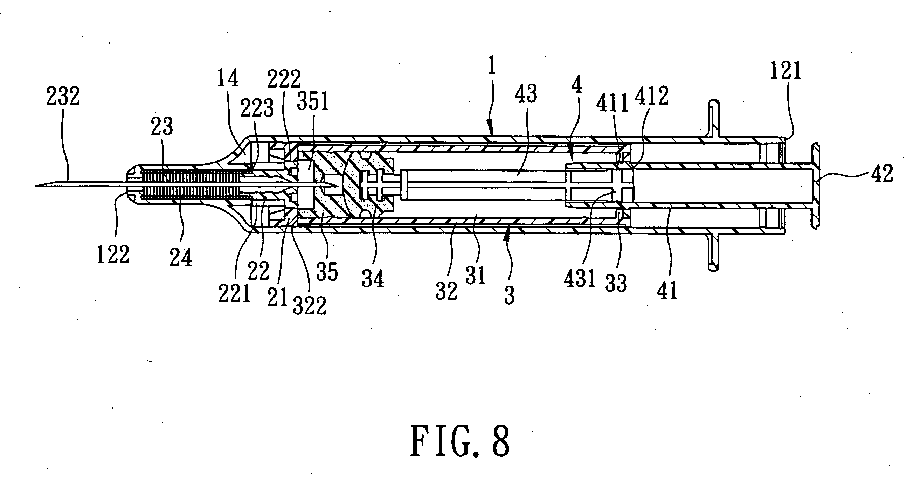

Fig. 8 is a sectional view of the first preferred embodiment after completion of the injection stroke;

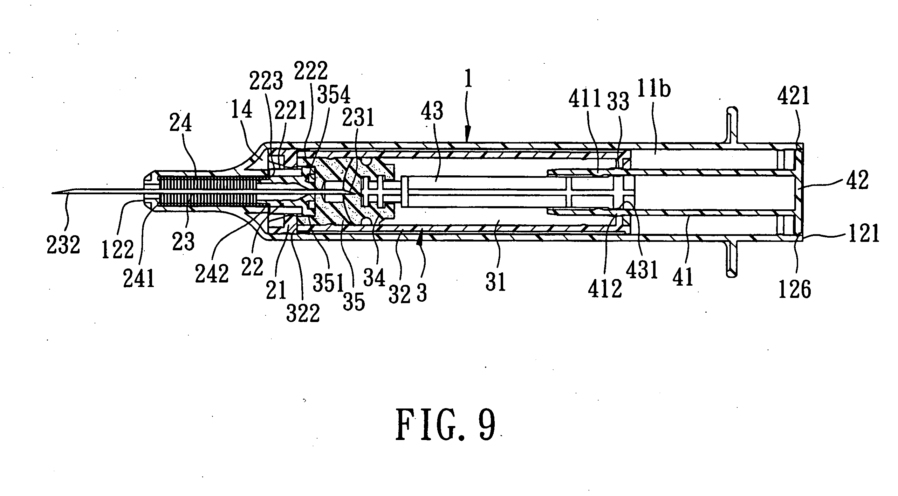

Fig. 9 is a sectional view of the first preferred embodiment, showing the state of disengagement of a seat member from a grip member;

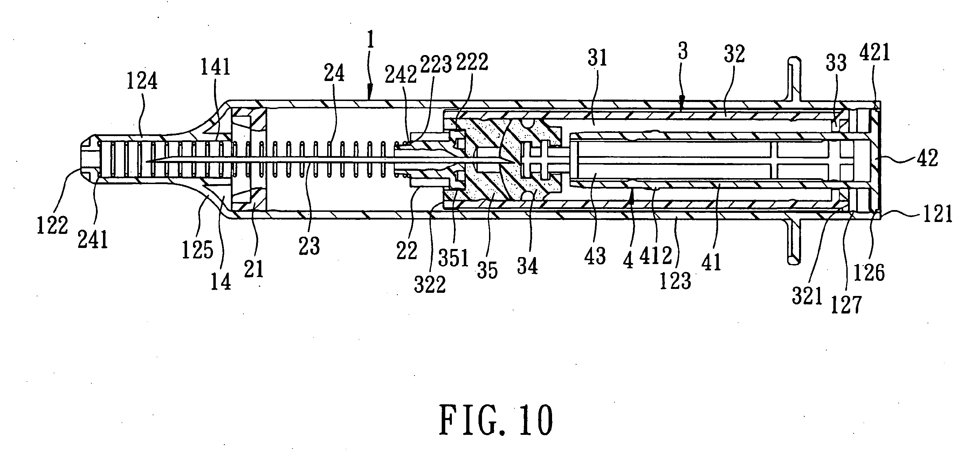

Fig. 10 is a sectional view of the first preferred embodiment, showing that a needle cannula is fully retracted into a barrel;

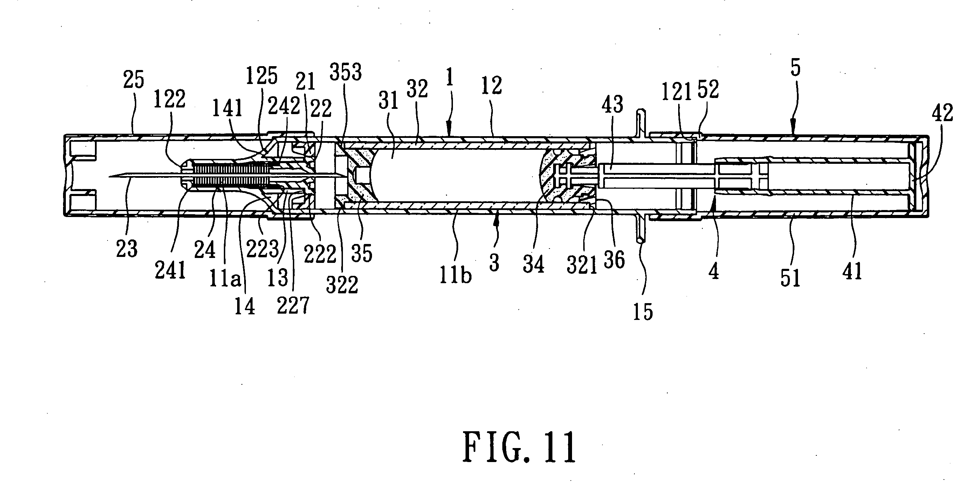

Fig. 11 is a sectional view of the second preferred embodiment of a disposable syringe according to this invention;

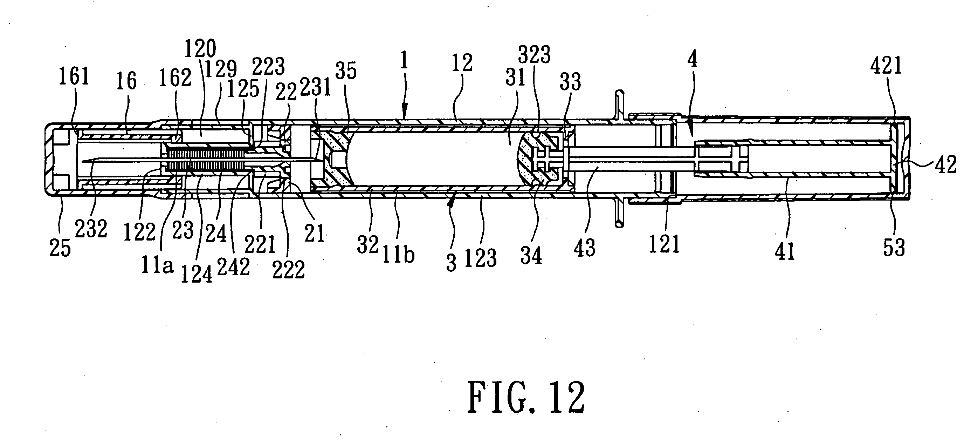

Fig. 12 is a sectional view of the third preferred embodiment of a disposable syringe according to this invention in the initial assembled position;

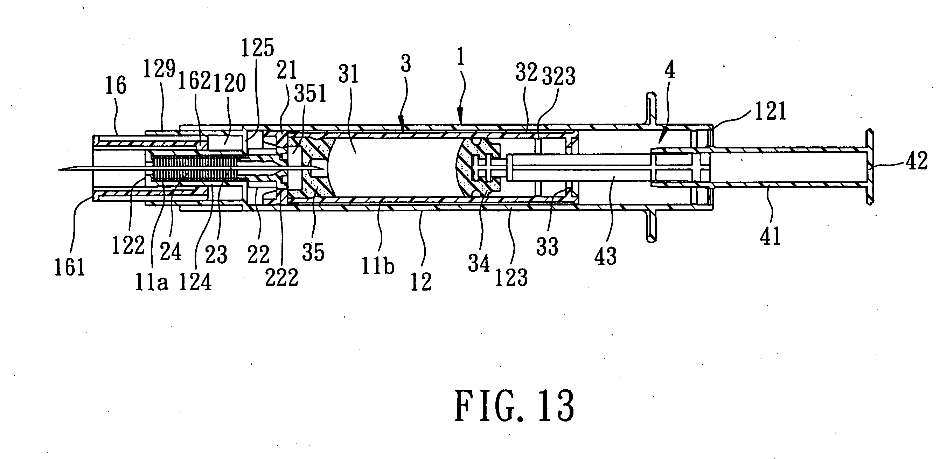

Fig. 13 is a sectional view of the third preferred embodiment during an injection stroke;

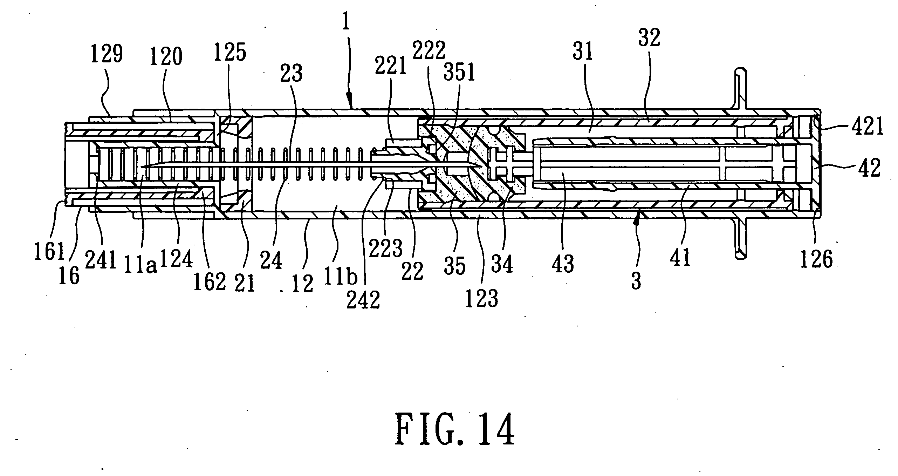

Fig. 14 is a sectional view of the third preferred embodiment, showing that a needle cannula is fully retracted into a barrel;

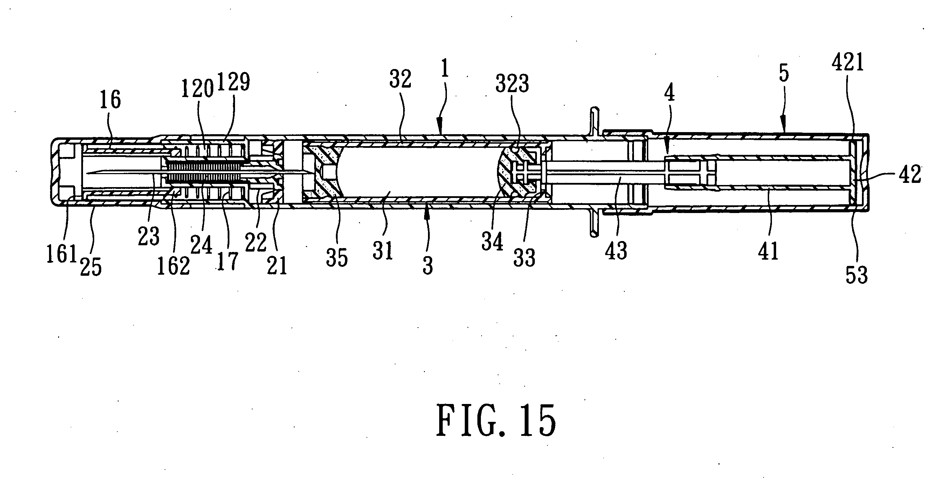

Figs. 15 to 18 respectively are sectional views of the fourth, fifth, sixth, and seventh preferred embodiments of a disposable syringe according to this invention;

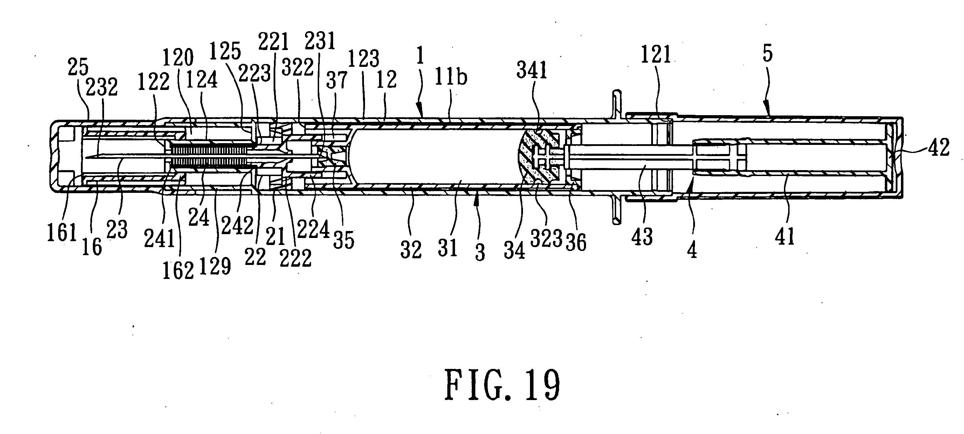

Fig. 19 is a sectional view of the eighth preferred embodiment of a disposable syringe according to this invention in the initial assembled position;

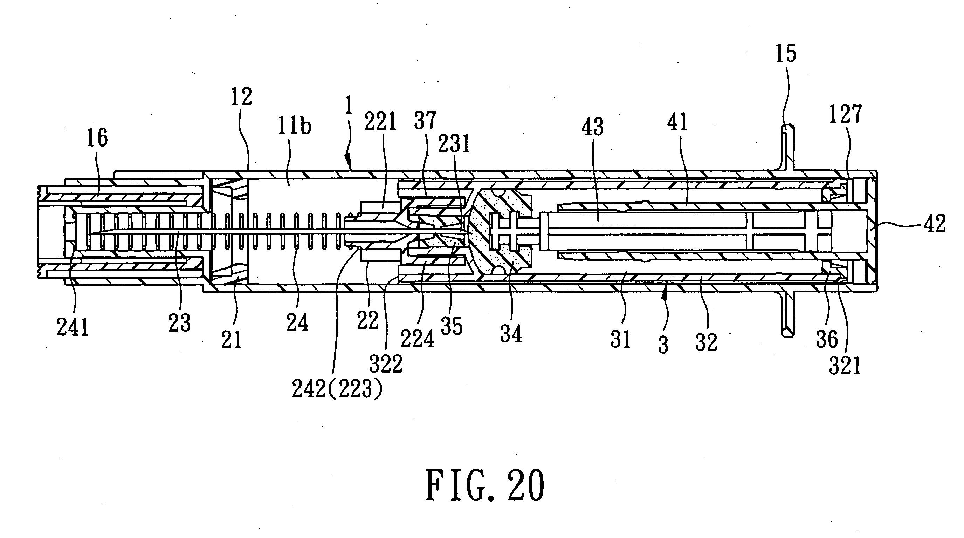

Fig. 20 is a sectional view of the eighth preferred embodiment, showing that a needle cannula is fully retracted into a barrel;

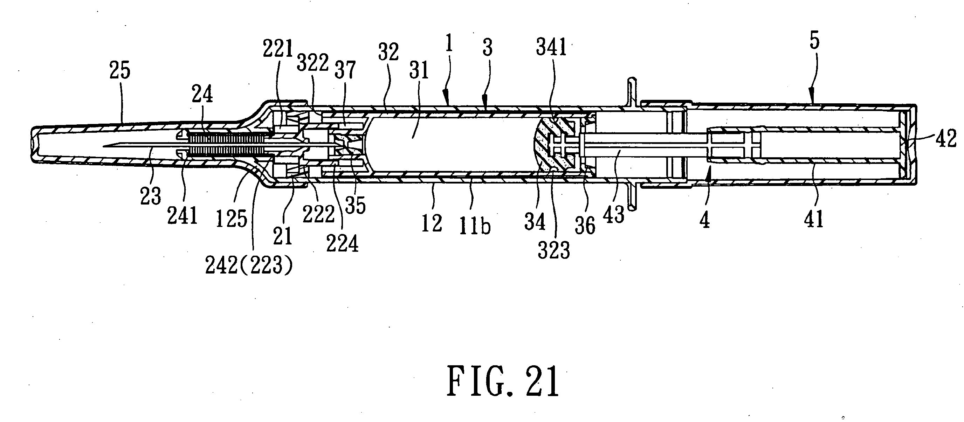

Fig. 21 is a sectional view of the ninth preferred embodiment of a disposable syringe according to this invention;

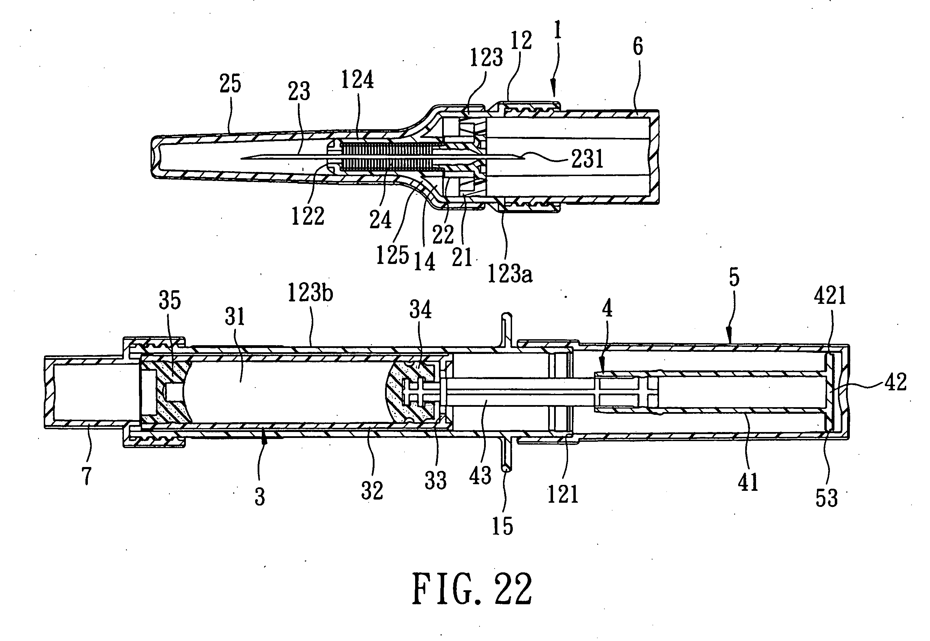

Fig. 22 is an exploded sectional view of the tenth preferred embodiment of a disposable syringe according to this invention;

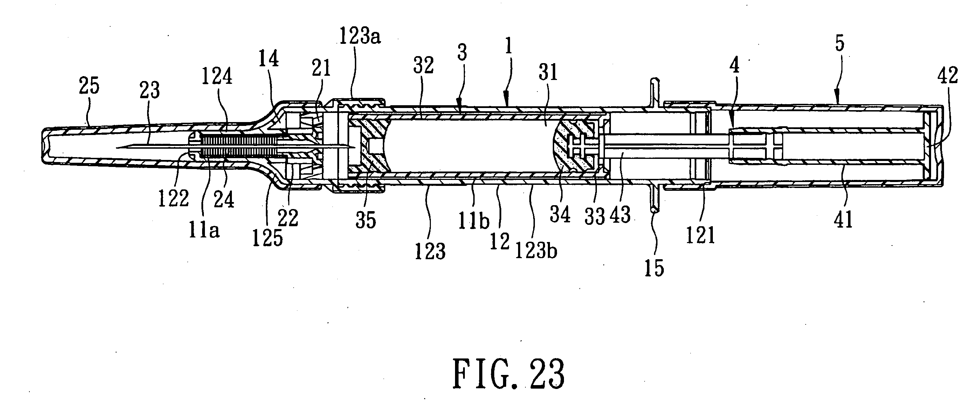

Fig. 23 is a sectional view of the tenth preferred embodiment in the initial assembled position;

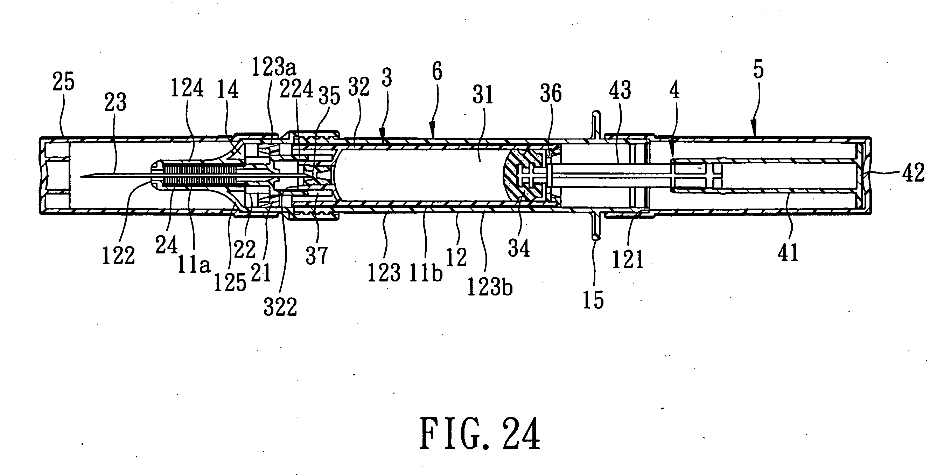

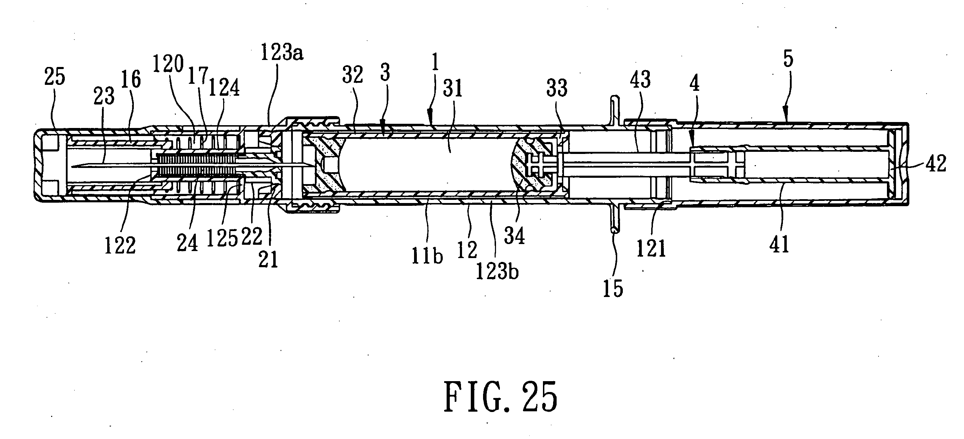

Figs. 24 and 25 respectively are sectional views of the eleventh and twelfth preferred embodiments of a disposable syringe according to this invention;

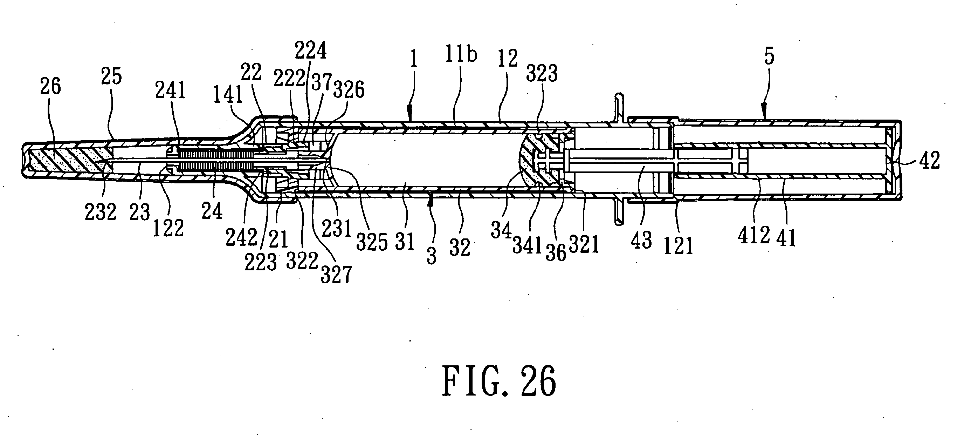

Fig. 26 is a sectional view of the thirteenth preferred embodiment of a disposable syringe according to this invention in the initial assembled position;

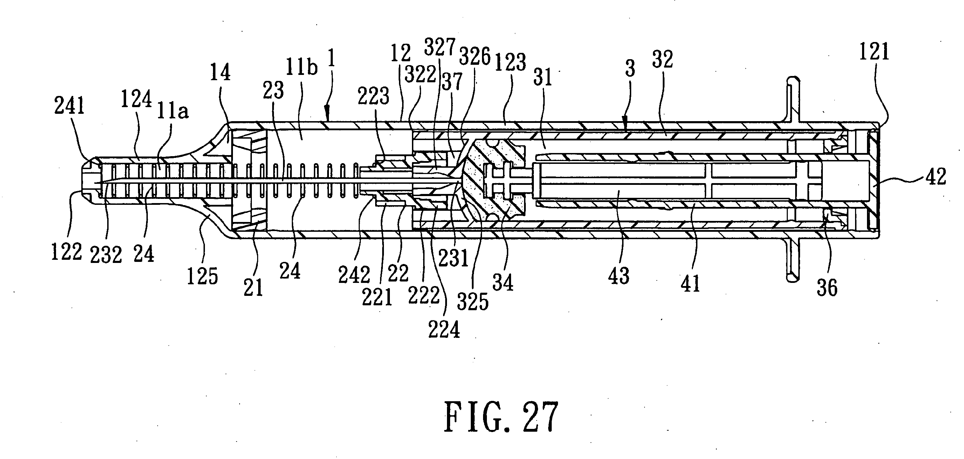

Fig. 27 is a sectional view of the thirteenth preferred embodiment, showing that a needle cannula is fully retracted into a barrel; and

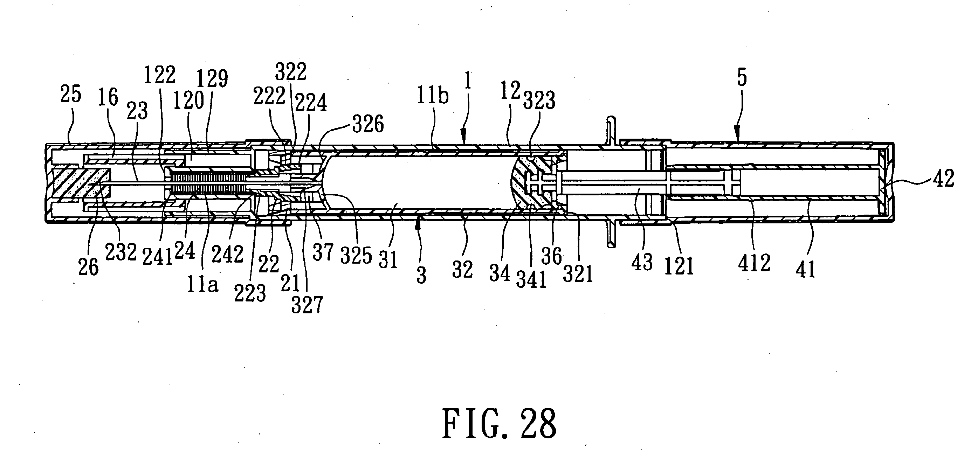

Fig. 28 is a sectional view of the fourteenth preferred embodiment of a disposable syringe according to this invention.

Before the present invention is described in greater detail, it should be noted that same reference numerals have been used to denote like elements throughout the specification.

Referring to Figs. 4 to 6, the first preferred embodiment of a disposable syringe according to the present invention is shown to comprise a barrel 1, a needle cannula 23, a seat member 22, a grip member 21, a biasing member 24, a tip protector 25, a carpule 3, a plunger 4, and a plunger protector 5.

The barrel 1 includes front and rear barrel ends 122, 121 opposite to each other along an axis in an axial direction, a surrounding barrel wall 12 which interconnects the front and rear barrel ends 122, 121, and which includes front and rear wall portions 124, 123 that are disposed proximate to the front and rear barrel ends 122,121 respectively, and that define front and rear compartments (11a,11b), respectively, and a finger flange 15 which is disposed proximate to the rear barrel end 121. In this embodiment, the rear compartment (11b) is larger than the front compartment (11a) in diameter so as to form a front annular shoulder 125 therebetween. The shoulder 125 has a plurality of fins 14 which extend towards the axis to terminate at an inner peripheral edge 141 that surrounds the axis. Moreover, a recess-like retaining portion 126 is disposed in the rear compartment (11b) adjacent to the rear barrel end 121. An annular stopper 127 is disposed forwardly of the retaining portion 126. A retaining ring 128 is disposed on the rear wall portion 123 adjacent to the front wall portion 124.

The needle cannula 23 has a tip end 232 which is disposed forwardly of the front barrel end 122 in a position of use (as shown in Fig. 5), and a communicating end 231 which is opposite to the tip end 232 along the axis and which is pointed.

The seat member 22 has a rear seat end surface 225 which holds the needle cannula 23, which permits rearward extension of the communicating end 231 of the needle cannula 23 therethrough, and which confronts rearwardly, a surrounding gripped portion 222 which surrounds the axis, and a surrounding front abutment surface 223 which is opposite to the rear seat end surface 225 and which confronts forwardly. The seat member 22 further has a tubular mount 226 which extends from the surrounding front abutment surface 223 into the front compartment (11a), and which is spaced apart from the inner peripheral edge 141 in radial directions by an inserted clearance that receives a lower end 242 of the biasing member 24 so as to permit the biasing member 24 to abut against the surrounding front abutment surface 223. The biasing member 24 is disposed in the front compartment (11a), and has an upper end 241 abutting against the front barrel end 122. The seat member 22 further has an annular stepped surface 227 which is spaced apart from the fins 14 of the shoulder 125 in the axial direction, and which extends radially and outwardly to join the gripped portion 222, and a plurality of fin spacers 221 which are angularly displaced from one another about the axis, and each of which is interposed between the annular stepped surface 227 and the fins 14 so as to brace the annular stepped surface 227 against the shoulder 125.

The grip member 21 is detachably retained on the rear wall portion 123 by a retaining ring 128, and is configured to be frictionally engaged with the gripped portion 222 so as to provide a resisting force that guards the gripped portion 222 against movement relative to the rear wall portion 123 in the axial direction during a piercing action of the needle cannula 23 for injection, and that permits disengagement of the grip member 21 from the gripped portion 222 so as to enable a subsequent movement of the seat member 22 when the grip member 21 is subjected to a pressing force.

The tip protector 25 is removably sleeved on the barrel wall 12 for shielding the needle cannula 23 in the position of use.

The carpule 3 has a tubular wall 32 which is configured to be fitted in the rear compartment (11b), and which extends in the axial direction to terminate at a tubular edge surface 322 and a rear wall end 321. The carpule 3 further has front and rear seal members 35, 34 which are spaced apart from each other in the axial direction, and which, in cooperation with the tubular wall 32, form a sealed chamber 31 for holding liquid medicament. The front seal member 35 is secured to the tubular wall 32 by means of inter-engagement of female and male engaging portions 352,324. The rear seal member 34 is limited to the tubular wall 32 by means of inter-engagement of female and male engaging portions 341,323 so as to guard against undesired movement of the rear seal member 34 before injection, and is forced forwardly to permit disengagement of the female engaging portion 341 from the male engaging portion 323 to be moved to abut against the front seal member 35 in an initial course so as to expel the liquid medicament out of the sealed chamber 31, thereby placing the carpule 3 in a used-up position, as shown in Fig. 8. The front seal member 35 has a mating surface 354 which is configured to have a cavity 351 that extends inwardly and towards the sealed chamber 31. Thus, when the carpule 3 is assembled in the rear compartment (11b), the pointed communicating end 231 of the needle cannula 23 can pierce the mating surface 354 of the front seal member 35 to be retained in and to pass through the mating surface 354 such that the needle cannula 23 is in fluid communication with the sealed chamber 31, as shown in Fig. 7. Moreover, the tubular wall 32 of the carpule 3 has a rear annular shoulder 33 which extends radially and inwardly from the rear wall end 321 to guard against a rearward movement of the rear seal member 34 relative to the tubular wall 32. Thus, once the rear seal member 34 abuts against the front seal member 35 in the used-up position, the pressing force is transmitted through the tubular wall 32 to force the tubular edge surface 322 against the grip member 21 in a subsequent course so as to effect disengagement of the grip member 21 from the gripped portion 222, thereby placing the seat member 22 in a disengaging position, as shown in Fig. 9, where the mating surface 354 is closer to the front barrel end 122 than when the carpule 3 is in the used-up position. At this time, the gripped portion 222 is received in the cavity 351 to ensure that the carpule 3 is kept together with the seat member 22.

The plunger 4 includes first and second plunger bodies 43,41. The first plunger body 43 has a push end 432 which is connected to the rear seal member 34 by virtue of a frictional force, and a first rear end portion 433 opposite to the push end 432 in the axial direction. The second plunger body 41 includes an operating end 42 which is disposed to extend outwardly of the rear barrel end 121 to be subjected to an external force (i.e., a pressing force applied by a user) that is greater in a subsequent course (i.e., a needle retraction stroke) than in an initial course (i.e., an injection stroke), and a second front end portion 413 which is opposite to the operating end 42, and which is releasably retained with the first rear end portion 433 by means of a mating engagement between an annular retaining protrusion 411 and an annular retaining groove 431 so as to generate a frictional force that is greater than the external force in the initial course and is smaller than the external force in the subsequent course. Moreover, the second plunger body 41 has an abutment 412 which is disposed on the second front end portion 413.

The plunger protector 5 is detachably sleeved on the rear wall portion 123 for shielding the plunger 4, and has a releasably engaging region 53 which is disposed on a peripheral wall 51 thereof and which is brought into frictional engagement with a periphery 421 of the operating end 42 of the plunger 4 when the plunger protector 5 is sleeved on the rear wall portion 123. The plunger protector 5 further has a step edge 52 which is disposed to abut against the rear barrel end 121 for positioning the plunger protector 5 in place.

Referring to Figs. 5 and 7, in the injection stroke, the tip and plunger protectors 25,5 are removed first to expose the needle cannula 23 and the plunger 4. The operator holds the finger flange 15 with his/her index and middle fingers and presses the operating end 42 of the plunger 4 forwardly with his/her thumb such that the carpule 3 is moved forwardly in the rear compartment (11b) to cause the communicating end 231 of the needle cannula 23 to pierce the mating surface 354 of the front seal member 35 so as to communicate the needle cannula 23 with the sealed chamber 31. The external pressing force is subsequently applied to the operating end 42 to cause the rear seal member 34 to be moved forwardly along the tubular wall 32 of the carpule 3 so as to place the carpule 3 in the used-up position, thereby completing the injection stroke, as shown in Fig. 8. When the carpule 3 is in the used-up position, the abutment 412 of the plunger 4 abuts against the rear annular shoulder 33 so as to impart the external force to the tubular wall 32 of the carpule 3.

As shown in Figs. 8 and 9, the external pressing force is subsequently applied to the operating end 42 to force the tubular edge surface 322 against the grip member 21 so as to effect disengagement of the grip member 21 from the gripped portion 222 resulting from blocking of the seat member 22 by the fins 14, thereby placing the seat member 22 in the disengaging position. In this embodiment, referring once again to Fig. 6, the front abutment surface 223 of the seat member 22 is spaced apart from the fins 14 by a small clearance in the position of use. The clearance can serve as a triggering space for facilitating a rearward movement of the seat member 22 after the seat member 22 is moved forwardly with the front seal member 35 to bring the front abutment surface 223 into abutment against the fins 14.

Once the grip member 21 is blocked by the fins 14, the external pressing force causes the gripped portion 222 of the seat member 22 to be received in the cavity 351 in the front seal member 35, further causes the disengagement of the retaining protrusion 411 of the second plunger body 41 from the retaining groove 431 in the first plunger body 43 to permit a forward movement of the second plunger body 41 relative to the first plunger body 43 until the periphery 421 of the operating end 42 is retained with the retaining portion 126 of the barrel 1 so as to guard against a rearward movement of the second plunger body 41 for preventing reuse of the syringe.

As shown in Fig. 10, when the seat member 22 is in the disengagingposition, the biasing force of thebiasingmember 24 is released to bias the seat member 22 together with the carpule 3 towards the rear barrel end 121 so as to retract the tip end 232 of the needle cannula 23 into the front compartment (11a), thereby placing the needle cannula 23 in a disposal position. The carpule 3 is stopped by the annular stopper 127. At the same time, a large part of the first plunger body 43 is retracted into the second plunger body 41.

Referring to Fig. 11, the second preferred embodiment of a disposable syringe according to this invention is shown to be similar to the previous embodiment in construction. In the second embodiment, the front seal member 35 has an annular flange 353 which is disposed forwardly of the tubular wall 32 and which is brought to abut against the tubular edge surface 322 so as to ensure fluid-tight engagement of the front seal member 35 with the tubular wall 32. An annular barrier 36 is disposed to abut against the rear wall end 321 of the carpule 3, and is detachably sleeved on the rear seal member 34 to serve as the rear annular shoulder. Moreover, a sleeve 13 extends rearwardly from the inner peripheral edge 141 of the front annular shoulder 125 such that the annular stepped surface 227 of the seat member 22 abuts against the sleeve 13 in the position of use.

Referring to Figs. 12 to 14, the third preferred embodiment of a disposable syringe according to this invention is shown to be similar to the first embodiment in construction. In the third embodiment, the barrel 1 has a tubular front wall 129 which extends forwardly from the rear wall portion 123 in the axial direction and which is spaced apart from the front wall portion 124 in radial directions to define a sliding channel 120 therebetween. A tubular touching member 16 is received in the sliding channel 120, and includes a tubular touching wall which surrounds the axis, and which has a rear engaging end 162 that is slidably engaged with the front wall portion 124, and a front touching end 161 that is movable by virtue of a sliding movement of the rear engaging end 162 between a forward position, where the touching end 161 is disposed forwardly of the tip end 232 of the needle cannula 23 for touching a patient's skin prior to piercing of the skin with the tip end 232, and a rearward position, where the touching end 161 is retracted to expose the tip end 232 for a piercing action. The front touching end 161 has a rough surface. The needle protector 25 removably covers the tubular front wall 129 so as to shield the needle cannula 23 before use.

During the injection stroke, the front touching end 161 is brought to touch a patient's skin and is forced to move rearwardly to the rearward position to expose the tip end 232 of the needle cannula 23 for the piercing action. When the touching member 16 comes into contact with the patient' s skin, the patient's attention to the needle cannula 23 is diverted thereto, thereby alleviating the pain caused by the pricking tip.

Referring to Fig. 15, the fourth preferred embodiment of a disposable syringe according to this invention is shown to be similar to the third embodiment in construction. In the fourth embodiment, the syringe further comprises a spring 17 which is disposed in the sliding channel 120 to bias the touching end 161 of the touching member 16 to the forward position.

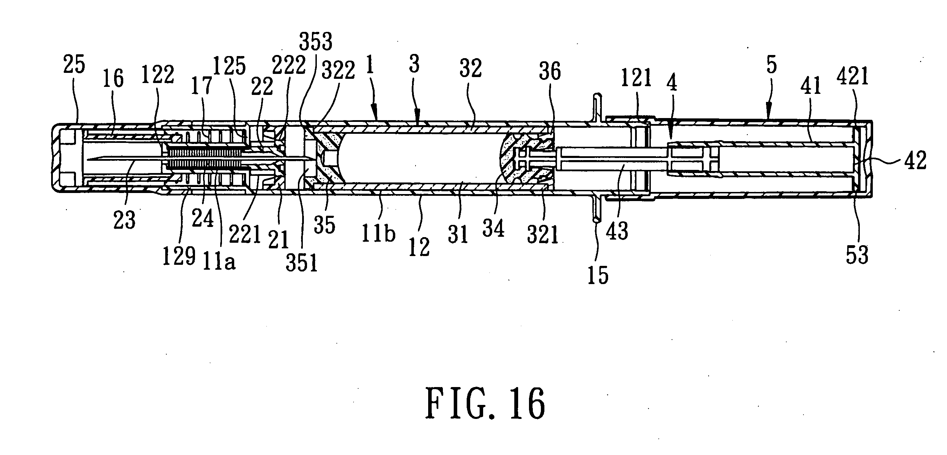

Referring to Fig. 16, the fifth preferred embodiment of a disposable syringe according to this invention is shown to be similar to the fourth embodiment in construction. In the fifth embodiment, the front seal member 35 has an annular flange 353 which is disposed forwardly of the tubular wall 32 and which is brought to abut against the tubular edge surface 322 so as to ensure fluid-tight engagement between the front seal member 35 and the tubular wall 32. An annular barrier 36 is disposed to abut against the rear wall end 321 of the carpule 3, and is detachably sleeved on the rear seal member 34 to serve as the rear annular shoulder.

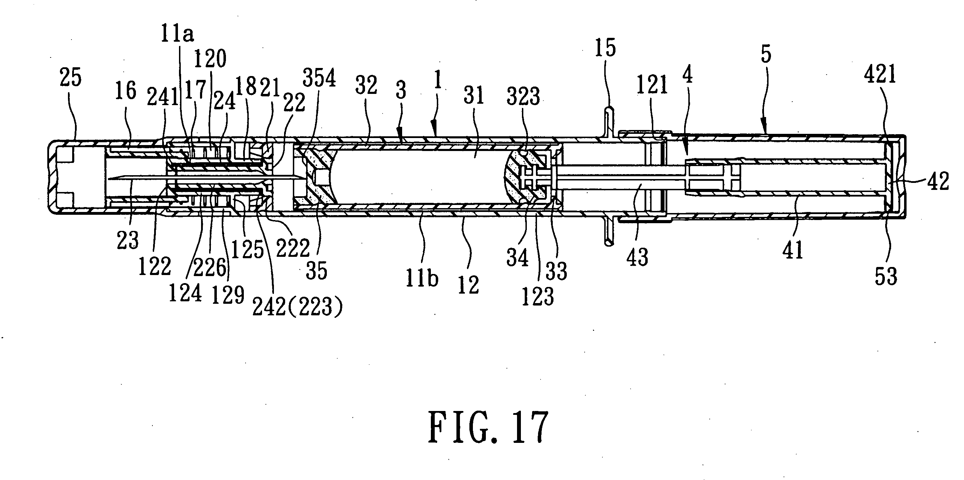

Referring to Fig. 17, the sixth preferred embodiment of a disposable syringe according to this invention is shown to be similar to the fourth embodiment in construction. In the sixth embodiment, the tubular mount 226 of the seat member 22 extends through the front compartment (11a) to abut against the front barrel end 122. The barrel 1 has a tubular spacer 18 which is integrally formed with and which extends rearwardly from the front annular shoulder 125 to permit abutment of the front abutment surface 223 of the seat member 22 thereagainst, and which encloses the lower end 242 of the biasing member 24 for steadying the biasing action of the biasing member 24.

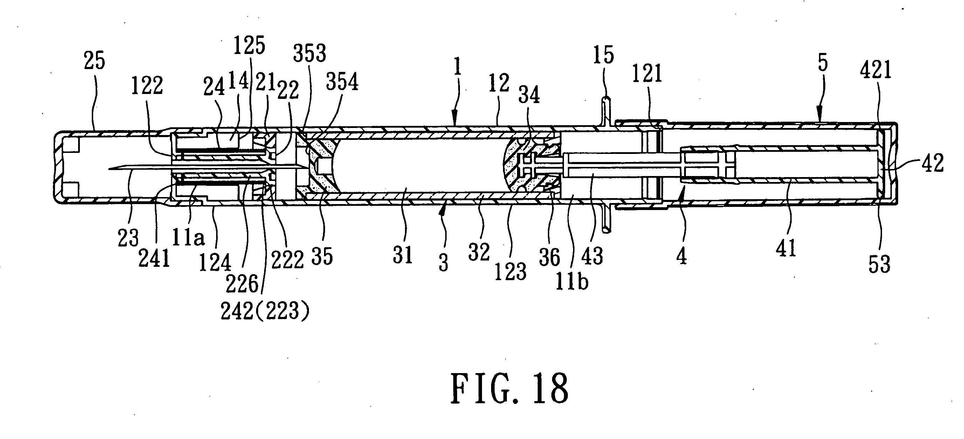

Referring to Fig. 18, the seventh preferred embodiment of a disposable syringe according to this invention is shown to be similar to the sixth embodiment in construction. In the seventh embodiment, the carpule 3 is the same as that of the second embodiment. The surrounding front abutment surface 223 of the seat member 22 is disposed to be spaced apart from the fins 14 of the front annular shoulder 125 so as to expose the lower end 242 of the biasing member 24. Thus, the tubular spacer 18 is omitted, and the biasing member 24 may have a larger dimension to enhance the biasing strength thereof.

Referring to Figs. 19 and 20, the eighth preferred embodiment of a disposable syringe according to this invention is shown to be similar to the third embodiment in construction. In the eighth embodiment, the carpule 3 has an annular keyslot 37 which is disposed adjacent to the tubular edge surface 322 and which extends in the axial direction to surround the front seal member 35. The seat member 22 has an annular key 224 which extends rearwardly from the gripped portion 222 and which is disposed to slide in and along the keyslot 37 for facilitating a forward movement of the carpule 3 along the axis when the seat member 22 is brought to the disengaging position. Besides, the engagement of the keyslot 37 with the key 224 facilitates rearward movement of the seat member 22 together with the carpule 3 toward the rear barrel end 121 so as to ensure retraction of the needle cannula 23 to the disposal position. By means of the provision of the keyslot 37 and the key 224, the carpule 3 can be firmly received in the rear compartment (11b), thereby preventing the communicating end 231 from accidentally piercing the front seal member 35 during transport.

Referring to Fig. 21, the ninth preferred embodiment of a disposable syringe according to this invention is shown to be similar to the eighth embodiment in construction, except that the barrel 1 is the same as that of the first embodiment.

Referring to Figs. 22 and 23, the tenth preferred embodiment of a disposable syringe according to this invention is shown to be similar to the first embodiment in construction. In the tenth embodiment, the barrel 1 has two parts. Particularly, the rear wall portion 123 of the barrel wall 12 includes a first wall segment (123a) which is integrally formed with and which extends rearwardly from the front wall portion 124 to receive the seat member 22 and the grip member 21 as a first unit, and a second wall segment (123b) which is threadedly engaged with the first wall segment (123a) to receive the carpule 3 and the plunger 4 as a second unit. A first end cap 6 is threadedly connected to the first wall segment (123a) for shielding the communicating end 231 of the needle cannula 23. A second end cap 7 is threadedly connected to the second wall segment (123b) for shielding the carpule 3. Thus, the first and second units can be packed separately to meet a variety of injection requirements.

Referring to Fig. 24, the eleventh preferred embodiment of a disposable syringe according to this invention is shown to be similar to the tenth embodiment in construction, except that the seat member 22 and the carpule 3 are the same as those of the ninth embodiment.

Referring to Fig. 25, the twelfth preferred embodiment of a disposable syringe according to this invention is shown to be similar to the tenth embodiment in construction, except that the front wall portion 124 of the barrel 1, the tubular touching member 16, and the spring 17 are the same as those of the fourth embodiment.

Referring to Figs. 26 and 27, the thirteenth preferred embodiment of a disposable syringe according to this invention is shown to be similar to the first embodiment in construction. In the thirteenth embodiment, the front seal member 325 of the carpule 3 is integrally formed with the tubular wall 32. Instead of the seat member 22 which holds the needle cannula 23, the carpule 3 has a tubular needle holding portion 327 which extends forwardly from the mating surface 326 along the axis and which holds the communicating end 231 of the needle cannula 23 to communicate the needle cannula 23 with the sealed chamber 31. The carpule 3 further has an annular keyslot 37 which extends forwardly from the mating surface 326 in the axial direction to surround the needle holding portion 327. The seat member 22 has an annular key 224 which extends rearwardly from the gripped portion 222 and which is disposed to slide in and along the keyslot 37. Moreover, an elastomeric packing 26 is stuffed in the tip protector 25 such that when the tip protector 25 is sleeved on the barrel wall 12, the tip end 232 of the needle cannula 23 is trapped in the elastomeric packing 26, thereby preventing fluid leakage.

Referringto Fig. 28, the fourteenthpreferredembodiment of a disposable syringe according to this invention is shown to be similar to the thirteenth embodiment in construction, except that the barrel 1 and the tubular touching member 16 are the same as those of the third embodiment.

As illustrated, the disposable syringe of this invention has the following advantages:
1. Since the communicating end 231 of the needle cannula 23 is held by the carpule 3 after the carpule 3 is mounted in the barrel 1, and since the seat member 22 is mated with the front seal member 35,325 when the seat member 22 is in the disengaging position, the seat member 22 can be moved together with the carpule 3 towards the rear barrel end 121 by the biasing action of the biasing member 24 in the needle retraction stroke, thereby ensuring the retraction of the needle cannula 23 into the barrel 1.
2. Since the carpule 3 can be pre-assembled in the barrel 1, clinical injection operation by health care workers is simplified and convenient to conduct. In addition, the syringe can be conveniently rendered unreusable after injection in a single operation.
3. Since the resisting force generated between the grip member 21 and the seat member 22, and the frictional force generated between the first and second plunger bodies 43,41 during pressing of the plunger 4 are overcome by the external pressing force, the injection and needle retraction strokes are smooth and successful.
4. Since the operating end 42 of the plunger 4 is retained with the retaining portion 126 of the barrel 1 to guard against a rearward movement of the plunger 4 after the operating end 42 is fully pressed in the barrel 1, reuse of the syringe can be prevented.

## Claims

1. A disposable syringe with a built-in carpule comprising:
a barrel (1) including front and rear barrel ends (122, 121) opposite to each other along an axis in an axial direction, a surrounding barrel wall (12) which interconnects said front and rear barrel ends (122,121), and which includes front and rear wall portions (124,123) that are disposed proximate to said front and rear barrel ends (122,121), respectively, and that define front and rear compartments (lla,llb), respectively;
a needle cannula (23) having a tip end (232) which is disposed forwardly of said front barrel end (122) in a position of use, and a communicating end (231) which is opposite to said tip end (232) along the axis;
a seat member (22) which has a surrounding gripped portion (222) surrounding the axis; and
a grip member (21) which is detachably retained on said rear wall portion (123), and which is configured to be frictionally engaged with said gripped portion (222) to provide a resisting force that guards said gripped portion (222) against movement relative to said rear wall portion (123) in the axial direction during a piercing action of said needle cannula (42) for injection, and that permits disengagement of said grip member (21) from said gripped portion (222) so as to enable a subsequent movement of said seat member (22) when said grip member (21) is subjected to a pressing force, **characterized by**:
a carpule (3) which has a tubular wall (32) that is configured to be fitted in said rear compartment (11b), and that extends towards said grip member (21) to terminate at a tubular edge surface (322), and front and rear seal members (35,34;325,34) that are spaced apart from each other in the axial direction, and that, in cooperation with said tubular wall (32), form a sealed chamber (31) for holding liquid medicament, said rear seal member (34) being disposed to be movable to abut against said front seal member (35,325) in an initial course so as to expel the liquid medicament out of said sealed chamber (31), thereby placing said carpule (3) in a used-up position, said front seal member (35,325) having a mating surface (354, 326) which is configured to permit said communicating end (231) to be retained with and to pass through said mating surface such that said needle cannula (23) is in fluid communication with said sealed chamber (31), and such that once said rear seal member (34) abuts against said front seal member (35, 325) in the used-up position, the pressing force is transmitted through said tubular wall (32) to force said tubular, edge surface (322) against said grip member (21) in a subsequent course so as to effect disengagement of said grip member (21) from said gripped portion (222), thereby placing said seat member (22) in a disengaging position, where said mating surface (354,326) is closer to said front barrel end (122) than that in the used-up position;
a plunger (4) which has a push end (432) that is disposed to engage and move said rear seal member (34) forwardly along said tubular wall (32) to bring said carpule (3) to the used-up position, and to move said mating surface (354) forwardly from the used-up position so as to permit said seat member (22) to be placed in the disengaging position, and an operating end (42) that is opposite to said push end (432) in the axial direction, and that is disposed to extend outwardly of said rear barrel end (121) to be subjected to an external force which is greater in the subsequent course than in the initial course; and
a biasing member (24) which is disposed in said front compartment (11a) such that, once said seat member (22) is in the disengaging position, said biasing member (24) biases said seat member (22) together with said carpule (3) towards said rear barrel end (121) to retract said tip end (232) of said needle cannula (23) into said barrel (1) , thereby placing said needle cannula (23) in a disposal position.

2. The disposable syringe with a built-in carpule of Claim 1, **characterized in that** said plunger (4) includes
a first plunger body (43) having said push end (432) which is disposed to engage and move with said rear seal member (34), and a first rear end portion (433) opposite to said push end (432), and
a second plunger body (41) including said operating end (42), and a second front end portion (413) which is opposite to said operating end (42), and which is releasably retained with said first rear end portion (433) by a frictional force that is greater than the external force in the initial course so as to permit said rear seal member (34) to be moved forwardly by the external force along said tubular wall (32) to thereby place said carpule (3) in the used-up position, and that is smaller than the external force in the subsequent course to permit said second plunger body (41) to move relative to said first plunger body (43) so as to impart the external force to said tubular wall (32) to thereby force said tubular edge surface (322) against said grip member (21) with the pressing force.

3. The disposable syringe with a built-in carpule of Claim 2, **characterized in that** said tubular wall (32) of said carpule (3) has a rear wall end (321) which is opposite to said tubular edge surface (322), and a rear annular shoulder (33,36) which extends radially and inwardly from said rear wall end (321), said second plunger body (41) having an abutment (412) which is disposed on said second front end portion (413) of said second plunger body (41) and which is configured to abut against said rear annular shoulder (33, 36) when said carpule (3) is in the used-up position so as to impart the external force to said tubular wall (32).

4. The disposable syringe with a built-in carpule of Claim 3, **characterized in that** said rear annular shoulder (33,36) is configured to guard against a rearward movement of said rear seal member (34) relative to said tubular wall (32).

5. The disposable syringe with a built-in carpule of Claim 2, **characterized in that** said barrel wall (12) has a retaining portion (126) adjacent to said rear barrel end (121), said second plunger body (41) being configured such that said operating end (42) is retained with said retaining portion (126) once said seat member (22) is placed in the disengaging position so as to guard against a rearward movement of said second plunger body (91).

6. The disposable syringe with a built-in carpule of Claim 2, **characterized in that** said first rear end portion (433) and said second front end portion (413) respectively have an annular retaining groove (431) and an annular retaining protrusion (411) which are matingly engaged with each other to generate the frictional force.

7. The disposable syringe with a built-in carpule of Claim 1, **characterized in that** said seat member (22) is disposed to fix said needle cannula (23) and to permit rearward extension of said communicating end (231) of said needle cannula (23) therethrough, said communicating end (231) being configured to be pointed so as to pierce said mating surface (354) of said front seal member (35) to thereby communicate said needle cannula (23) with said sealed chamber (31).

8. The disposable syringe with a built-in carpule of Claim 7, **characterized in that** said mating surface (354) of said front seal member (35) has a cavity (351) which extends inwardly and towards said sealed chamber (31) so as to receive said gripped portion (222) of said seat member (22) when said seat member (22) is in the disengaging position, thereby ensuring that said carpule (3) is kept together with said seat member (22) when said seat member (22) is biased towards said rear barrel end (121).

9. The disposable syringe with a built-in carpule of Claim 7, **characterized in that** said front seal member (35) has an annular flange (353) which is disposed forwardly of said tubular wall (32) and which is brought to abut against said tubular edge surface (322) so as to ensure fluid-tight engagement between said front seal member (35) and said tubular wall (32).

10. The disposable syringe with a built-in carpule of Claim 7, **characterized in that** said rear wall portion (123) includes a first wall segment (123a) which is integrally formed with and which extends rearwardly from said front wall portion (124) to receive said seat member (22) and said grip member (21), and a second wall segment (123b) which is detachably coupled to said first wall segment (123a) to receive said carpule (3) and said plunger (4).

11. The disposable syringe with a built-in carpule of Claim 10, further **characterized by** a first end cap (6) which is connected to said first wall segment (123a) for shielding said communicating end (231) of said needle cannula (23), and a second end cap (7) which is connected to said second wall segment (123b) for shielding said carpule (3).

12. The disposable syringe with a built-in carpule of Claim 1, **characterized in that** said carpule (3) has a tubular needle holding portion (327) which extends forwardly from said mating surface (326) along the axis and which holds said communicating end (231) of said needle cannula (23) to communicate said needle cannula (23) with said sealed chamber (31).

13. The disposable syringe with a built-in carpule of Claim 12, further **characterized by** a tip protector (25) which is removably sleeved on said barrel wall (12) for shielding said needle cannula (23), and an elastomeric packing (26) which is stuffed in said tip protector (25) such that when said tip protector is sleeved on said barrel wall (12), said tip end (232) of said needle cannula (23) is trapped in said elastomeric packing (26).

14. The disposable syringe with a built-in carpule of Claim 1, **characterized in that** said rear compartment (11b) is larger than said front compartment (11a) in diameter so as to form a front annular shoulder (125) therebetween, which extends towards the axis and terminates at an inner peripheral edge (141) that surrounds said needle cannula (23).

15. The disposable syringe with a built-in carpule of Claim 14, **characterized in that** said seat member (22) includes a surrounding front abutment surface (223) which is disposed to confront said front compartment (11a), and a rear seat end surface (225) which is opposite to said surrounding front abutment surface (223), and which confronts said mating surface (354) to hold said communicating end (231) steadily so as to facilitate passage of said communicating end (231) through said mating surface (354).

16. The disposable syringe with a built-in carpule of Claim 15, **characterized in that** said seat member (22) includes a tubular mount (226) which extends from said surrounding front abutment surface (223) into said front compartment (11a), and which is spaced apart from said inner peripheral edge (141) in radial directions by an inserted clearance that receives a lower end (242) of said biasing member (24) so as to permit said biasing member (24) to abut against said surrounding front abutment surface (223).

17. The disposable syringe with a built-in carpule of Claim 16, **characterized in that** said tubular mount (226) extends through said front compartment (11a) to engage with said front barrel end (122).

18. The disposable syringe with a built-in carpule of Claim 17, **characterized in that** said surrounding front abutment surface (223) is disposed to be spaced apart from said front annular shoulder (125) such that said lower end (242) of said biasing member (24) is exposed.

19. The disposable syringe with a built-in carpule of Claim 18, **characterized in that** said barrel (1) has a tubular spacer (18) which is disposed between said front annular shoulder (125) and said surrounding front abutment surface (223) of said seat member (22), and which encloses said exposed lower end (242) of said biasing member (24), said tubular spacer (18) being conf igured to be integrally formed with said front annular shoulder (125).

20. The disposable syringe with a built-in carpule of Claim 16, **characterized in that** said seat member (22) includes an annular stepped surface (227) which is spaced apart from said front annular shoulder (125) in the axial direction, and which extends radially and outwardly to join said surrounding gripped portion (222), and a plurality of fin spacers (221) which are angularly displaced from one another about the axis, and each of which is interposed between said annular stepped surface (227) and said front annular shoulder (125) so as to brace said annular stepped surface (227) against said front annular shoulder (125).

21. The disposable syringe with a built-in carpule of Claim 1, **characterized in that** said carpule (3) has a keyslot (37) which is disposed adjacent to said tubular edge surface (322) and which extends in the axial direction, said seat member (22) having a key (224) which extends rearwardly from said gripped portion (222) and which is disposed to slide in and along said keyslot (37).

22. The disposable syringe with a built-in carpule of Claim 1, further **characterized by** a tubular touching member (16) that includes a tubular touching wall which surrounds the axis, and which has a rear engaging end (162) that is slideably engaged with said front wall portion (124), and a front touching end (161) that is movable by virtue of a sliding movement of said rear engaging end (162) between a forward position, where said touching end (161) is disposed forwardly of said tip end (232) of said needle cannula (23) for touching a patient's skin prior to piercing the patient's skin with said tip end (232) , and a rearward position, where said touching end (161) is retracted to expose said tip end (232) for a piercing action.

23. The disposable syringe with a built-in carpule of Claim 22, further **characterized by** a spring (17) which is disposed to bias said touching end (161) to the forward position.

24. The disposable syringe with a built-in carpule of Claim 22, **characterized in that** said barrel (1) has a tubular front wall (129) which extends forwardly from said rear wall portion (123) in the axial direction and which is spaced apart from said front wall portion (124) in radial directions todefine a sliding channel (120) therebetween, said sliding channel (120) being configured to receive said touching member (16) and guide the movement of said rear engaging end (162) relative thereto, said syringe further comprising a needle protector (25) which is configured to removably cover said tubular front wall (129) so as to shield said needle cannula (23).

25. The disposable syringe with abuilt-in carpule of Claim 1, further **characterized by** a plunger protector (5) which is detachably sleeved on said rear wall portion (123) for shielding said plunger (4), and which has a releasably engaging region (53) that is brought into frictional engagement with said operating end (42) of said plunger (4) when said plunger protector (5) is sleeved on said rear wall portion (123).

## Patentansprüche

1. Einwegspritze mit einer eingebauten Zylinderampulle, wobei die Spritze Folgendes umfasst:
einen Zylinder (1), der ein vorderes und ein hinteres Zylinderende (122, 121), die einander längs einer Achse in einer axialen Richtung gegenüberliegen, eine umschließende Zylinderwand (12), die das vordere und das hintere Zylinderende (122, 121) miteinander verbindet und die einen vorderen und einen hinteren Wandabschnitt (124, 123) einschließt, die nahe dem vorderen bzw. dem hinteren Zylinderende (122, 121) angeordnet sind und die eine vordere bzw. eine hintere Abteilung (11a, 11b) definieren, einschließt,
eine Nadelkanüle (23), die ein Spitzenende (232), das in einer Verwendungsstellung vor dem vorderen Zylinderende (122) angeordnet ist, und ein Verbindungsende (231), das dem Spitzenende (232) längs der Achse gegenüberliegt, hat,
ein Sitzelement (22), das einen umschließenden ergriffenen Abschnitt (222), der die Achse umschließt, hat, und
ein Griffelement (21), das abnehmbar an dem hinteren Wandabschnitt (123) festgehalten wird und das dafür konfiguriert ist, reibschlüssig mit dem ergriffenen Abschnitt (222) in Eingriff gebracht zu werden, um eine Widerstandskraft bereitzustellen, die während eines Durchstechvorgangs der Nadelkanüle (23) zum Injizieren den ergriffenen Abschnitt (222) gegen eine Bewegung im Verhältnis zu dem hinteren Wandabschnitt (123) in der axialen Richtung schützt und die ein Ausrücken des Griffelements (21) von dem ergriffenen Abschnitt (222) ermöglicht, um so eine anschließende Bewegung des Sitzelements (22) zu ermöglichen, wenn das Griffelement (21) einer Druckkraft ausgesetzt wird, **gekennzeichnet durch**:
eine Zylinderampulle (3), die eine röhrenförmige Wand (32), die dafür konfiguriert ist, in der hinteren Abteilung (11b) angebracht zu werden, und die sich zu dem Griffelement (21) hin erstreckt, um an einer röhrenförmigen Kantenfläche (322) zu enden, und ein vorderes und ein hinteres Dichtungselement (35, 34; 325, 34), die mit Zwischenraum zueinander in der axialen Richtung angeordnet sind und die, in Zusammenwirken mit der röhrenförmigen Wand (32), eine abgedichtete Kammer (31) zum Aufnehmen eines flüssigen Medikaments bilden, hat, wobei das hintere Dichtungselement (34) dafür angeordnet ist, beweglich zu sein, um in einem anfänglichen Gang an das vordere Dichtungselement (35, 325) anzustoßen, um so das flüssige Medikament aus der abgedichteten Kammer (31) auszustoßen, wodurch die Zylinderampulle (3) in eine aufgebrauchte Stellung gebracht wird, wobei das vordere Dichtungselement (35, 325) eine Passfläche (354, 326) hat, die dafür konfiguriert ist, zu ermöglichen, dass das Verbindungsende (231) mit der Passfläche festgehalten wird und **durch** dieselbe hindurchgeht derart, dass die Nadelkanüle (23) in Fluidverbindung mit der abgedichteten Kammer (31) steht, und derart, dass, sobald das hintere Dichtungselement (34) in der aufgebrauchten Stellung an das vordere Dichtungselement (35, 325) anstößt, die Druckkraft **durch** die röhrenförmige Wand (32) weitergeleitet wird, um die röhrenförmige Kantenfläche (322) in einem anschließenden Gang gegen das Griffelement (21) zu drängen, um so ein Ausrücken des Griffelements (21) von dem ergriffenen Abschnitt (222) zu bewirken, um **dadurch** das Sitzelement (22) in eine Ausrückstellung zu bringen, wobei sich die Passfläche (354, 326) näher an dem vorderen Zylinderende (122) befindet als in der aufgebrauchten Stellung,
einen Druckkolben (4), der ein Druckende (432), das dafür angeordnet ist, das hintere Dichtungselement (34) in Eingriff zu nehmen und längs der röhrenförmigen Wand (32) nach vom zu bewegen, um die Zylinderampulle (3) zu der aufgebrauchten Stellung zu bringen und um die Passfläche (354) von der aufgebrauchten Stellung nach vom zu bewegen, um so zu ermöglichen, dass das Sitzelement (22) in die Ausrückstellung gebracht wird, und ein Betätigungsende (42) hat, das dem Druckende (432) in der axialen Richtung gegenüberliegt und das dafür angeordnet ist, sich aus dem hinteren Zylinderende (121) heraus zu erstrecken, um einer äußeren Kraft ausgesetzt zu werden, die in dem anschließenden Gang größer ist als in dem anfänglichen Gang, und
ein Vorspannelement (24), das in der vorderen Abteilung (11a) angeordnet ist derart, dass, sobald sich das Sitzelement (22) in der Ausrückstellung befindet, das Vorspannelement (24) das Sitzelement (22) zusammen mit der Zylinderampulle (3) zu dem hinteren Zylinderende (121) hin vorspannt, um das Spitzenende (232) der Nadelkanüle (23) in den Zylinder (1) einzuziehen, wodurch die Nadelkanüle (23) in eine Entsorgungsstellung gebracht wird.

2. Einwegspritze mit einer eingebauten Zylinderampulle nach Anspruch 1, **dadurch gekennzeichnet, dass** der Druckkolben (4) Folgendes einschließt:
einen ersten Druckkolbenkörper (43), der das Druckende (432), das dafür angeordnet ist, das hintere Dichtungselement (34) in Eingriff zu nehmen und sich mit demselben zu bewegen, und einen ersten, hinteren Endabschnitt (433) gegenüber dem Druckende (432) hat, und
einen zweiten Druckkolbenkörper (41), der das Betätigungsende (42) und einen zweiten, vorderen Endabschnitt (413) einschließt, der dem Betätigungsende (42) gegenüberliegt und der lösbar mit dem ersten, hinteren Endabschnitt (433) festgehalten wird, durch eine Reibungskraft, die größer ist als die äußere Kraft in dem anfänglichen Gang, um so zu ermöglichen, dass das hintere Abdichtungselement (34) durch die äußere Kraft längs der röhrenförmigen Wand (32) nach vom bewegt wird, um dadurch die Zylinderampulle (3) in die aufgebrauchte Stellung zu bringen, und die kleiner ist als die äußere Kraft in dem anschließenden Gang, um so zu ermöglichen, dass sich der zweite Druckkolbenkörper (41) im Verhältnis zu dem ersten Druckkolbenkörper (43) bewegt, um so die äußere Kraft der röhrenförmigen Wand (32) mitzuteilen, um dadurch die röhrenförmige Kantenfläche (322) mit der Druckkraft gegen das Griffelement (21) zu drängen.

3. Einwegspritze mit einer eingebauten Zylinderampulle nach Anspruch 2, **dadurch gekennzeichnet, dass** die röhrenförmige Wand (32) der Zylinderampulle (3) ein hinteres Wandende (321), das der röhrenförmigen Kantenfläche (322) gegenüberliegt, und einen hinteren ringförmigen Absatz (33, 36), der sich in Radialrichtung und von dem hinteren Wandende (321) nach innen erstreckt, hat, wobei der zweite Druckkolbenkörper (41) ein Widerlager (412) hat, das an dem zweiten, vorderen Endabschnitt (413) des zweiten Druckkolbenkörpers (41) angeordnet ist und das dafür konfiguriert ist, an den hinteren ringförmigen Absatz (33, 36) anzustoßen, wenn sich die Zylinderampulle (3) in der aufgebrauchten Stellung befindet, um so die äußere Kraft der röhrenförmigen Wand (32) mitzuteilen.

4. Einwegspritze mit einer eingebauten Zylinderampulle nach Anspruch 3, **dadurch gekennzeichnet, dass** der hintere ringförmige Absatz (33, 36) dafür konfiguriert ist, gegen eine Rückwärtsbewegung des hinteren Dichtungselements (34) im Verhältnis zu der röhrenförmigen Wand (32) zu schützen.

5. Einwegspritze mit einer eingebauten Zylinderampulle nach Anspruch 2, **dadurch gekennzeichnet, dass** die Zylinderwand (12) einen Festhalteabschnitt (126) angrenzend an das hintere Zylinderende (121) hat, wobei der zweite Druckkolbenkörper (41) derart konfiguriert ist, dass das Betätigungsende (42) mit dem Festhalteabschnitt (126) festgehalten wird, sobald das Sitzelement (22) in die Ausrückstellung gebracht ist, um so gegen eine Rückwärtsbewegung des zweiten Druckkolbenkörpers (41) zu schützen.

6. Einwegspritze mit einer eingebauten Zylinderampulle nach Anspruch 2, **dadurch gekennzeichnet, dass** der erste, hintere Endabschnitt (433) bzw. der zweite, vordere Endabschnitt (413) eine ringförmige Festhalterille (431) und einen ringförmigen Festhaltevorsprung (411) haben, die passend miteinander in Eingriff gebracht werden, um die Reibungskraft zu erzeugen.

7. Einwegspritze mit einer eingebauten Zylinderampulle nach Anspruch 1, **dadurch gekennzeichnet, dass** das Sitzelement (22) dafür angeordnet ist, die Nadelkanüle (23) zu fixieren und eine Rückwärtsausdehnung des Verbindungsendes (231) der Nadelkanüle (23) durch dasselbe zu ermöglichen, wobei das Verbindungsende (231) so konfiguriert ist, dass es zugespitzt ist, um so die Passfläche (354) des vorderen Dichtungselements (35) zu durchstechen, um dadurch die Nadelkanüle (23) in Verbindung mit der abgedichteten Kammer (31) zu setzen.

8. Einwegspritze mit einer eingebauten Zylinderampulle nach Anspruch 7, **dadurch gekennzeichnet, dass** die Passfläche (354) des vorderen Dichtungselements (35) einen Hohlraum (351) hat, der sich nach innen und zu der abgedichteten Kammer (31) hin erstreckt, um so den ergriffenen Abschnitt (222) des Sitzelements (22) aufzunehmen, wenn sich das Sitzelement (22) in der Ausrückstellung befindet, wodurch sichergestellt wird, dass die Zylinderampulle (3) mit dem Sitzelement (22) zusammengehalten wird, wenn das Sitzelement (22) zu dem hinteren Zylinderende (121) hin vorgespannt wird.

9. Einwegspritze mit einer eingebauten Zylinderampulle nach Anspruch 7, **dadurch gekennzeichnet, dass** das vordere Dichtungselement (35) einen ringförmigen Flansch (353) hat, der vor der röhrenförmigen Wand (32) angeordnet ist und der dazu gebracht wird, an die röhrenförmige Kantenfläche (322) anzustoßen, um so einen fluiddichten Eingriff zwischen dem vorderen Abdichtungselement (35) und der röhrenförmigen Wand (32) sicherzustellen.

10. Einwegspritze mit einer eingebauten Zylinderampulle nach Anspruch 7, **dadurch gekennzeichnet, dass** der hintere Wandabschnitt (123) ein erstes Wandsegment (123a), das integral mit dem vorderen Wandabschnitt (124) geformt ist und sich von dem vorderen Wandabschnitt (124) nach hinten erstreckt, um das Sitzelement (22) und das Griffelement (21) aufzunehmen, und ein zweites Wandsegment (123b), das abnehmbar an das erste Wandsegment (123a) gekoppelt ist, um die Zylinderampulle (3) und den Druckkolben (4) aufzunehmen, einschließt.

11. Einwegspritze mit einer eingebauten Zylinderampulle nach Anspruch 10, ferner **gekennzeichnet durch** eine erste Endkappe (6), die mit dem ersten Wandsegment (123a) verbunden ist, um das Verbindungsende (231) der Nadelkanüle (23) zu schützen, und eine zweite Endkappe (7), die mit dem zweiten Wandsegment (123b) verbunden ist, um die Zylinderampulle (3) zu schützen.

12. Einwegspritze mit einer eingebauten Zylinderampulle nach Anspruch 1, **dadurch gekennzeichnet, dass** die Zylinderampulle (3) einen röhrenförmigen Nadelhalteabschnitt (327) hat, der sich von der Passfläche (326) entlang der Achse nach vom erstreckt und der das Verbindungsende (231) der Nadelkanüle (23) hält, um die Nadelkanüle (23) mit der abgedichteten Kammer (31) in Verbindung zu setzen.

13. Einwegspritze mit einer eingebauten Zylinderampulle nach Anspruch 12, ferner **gekennzeichnet durch** einen Spitzenschützer (25), der abnehmbar auf die Zylinderwand (12) geschoben wird, um die Nadelkanüle (23) zu schützen, und einen Elastomer-Füllkörper (26), der in den Spitzenschützer (25) gestopft ist derart, dass, wenn der Spitzenschützer auf die Zylinderwand (12) geschoben wird, das Spitzenende (232) der Nadelkanüle (23) in dem Elastomer-Füllkörper (26) eingeschlossen wird.

14. Einwegspritze mit einer eingebauten Zylinderampulle nach Anspruch 1, **dadurch gekennzeichnet, dass** die hintere Abteilung (11a) im Durchmesser größer ist als die vordere Abteilung (11a), um so einen vorderen ringförmigen Absatz (125) zwischen denselben zu bilden, der sich zu der Achse hin erstreckt und an einer Innenumfangskante (141) endet, welche die Nadelkanüle (23) umschließt.

15. Einwegspritze mit einer eingebauten Zylinderampulle nach Anspruch 14, **dadurch gekennzeichnet, dass** das Sitzelement (22) eine umschließende vordere Widerlagerfläche (223), die dafür angeordnet ist, der vorderen Abteilung (11a) gegenüberzustehen, und eine hintere Sitzendfläche (225) einschließt, die der umschließenden vorderen Widerlagerfläche (223) gegenüberliegt und die der Passfläche (354) gegenübersteht, um das Verbindungsende (231) unbeweglich zu halten, um so den Durchgang des Verbindungsendes (231) durch die Passfläche (354) zu erleichtern.

16. Einwegspritze mit einer eingebauten Zylinderampulle nach Anspruch 15, **dadurch gekennzeichnet, dass** das Sitzelement (22) eine röhrenförmige Halterung (226) einschließt, die sich von der umschließenden vorderen Widerlagerfläche (223) in die vordere Abteilung (11a) erstreckt und die durch eine eingesetzte Aussparung, die ein unteres Ende (242) des Vorspannelements (24) aufnimmt, in radialen Richtungen mit Abstand von der Innenumfangskante (141) angeordnet ist, um so zu ermöglichen, dass das Vorspannelement (24) an die umschließende vordere Widerlagerfläche (223) anstößt.

17. Einwegspritze mit einer eingebauten Zylinderampulle nach Anspruch 16, **dadurch gekennzeichnet, dass** sich die röhrenförmige Halterung (226) durch die vordere Abteilung (11a) erstreckt, um mit dem vorderen Zylinderende (122) ineinanderzugreifen.

18. Einwegspritze mit einer eingebauten Zylinderampulle nach Anspruch 17, **dadurch gekennzeichnet, dass** die umschließende vordere Widerlagerfläche (223) so angeordnet ist, dass sie mit Abstand entfernt von dem vorderen ringförmigen Absatz (125) angeordnet ist derart, dass das untere Ende (242) des Vorspannelements (24) freigelegt ist.

19. Einwegspritze mit einer eingebauten Zylinderampulle nach Anspruch 18, **dadurch gekennzeichnet, dass** der Zylinder (1) ein röhrenförmiges Abstandsstück (18) hat, das zwischen dem vorderen ringförmigen Absatz (125) und der umschließenden vorderen Widerlagerfläche (223) des Sitzelements (22) angeordnet ist und welches das freigelegte untere Ende (242) des Vorspannelements (24) einschließt, wobei das röhrenförmige Abstandsstück (18) so konfiguriert ist, dass es integral mit dem vorderen ringförmigen Absatz (125) geformt ist.

20. Einwegspritze mit einer eingebauten Zylinderampulle nach Anspruch 16, **dadurch gekennzeichnet, dass** das Sitzelement (22) eine ringförmige abgestufte Fläche (227), die mit Abstand in der axialen Richtung entfernt von dem vorderen ringförmigen Absatz (125) angeordnet ist und die sich in Radialrichtung und nach außen erstreckt, um sich mit dem umgebenden ergriffenen Abschnitt (222) zu verbinden, und mehrere Rippenabstandsstücke (221) einschließt, die winklig um die Achse voneinander versetzt sind und deren jedes zwischen die ringförmige abgestufte Fläche (227) und den vorderen ringförmigen Absatz (125) geschaltet ist, um so die ringförmige abgestufte Fläche (227) gegen den vorderen ringförmigen Absatz (125) zu verspannen.

21. Einwegspritze mit einer eingebauten Zylinderampulle nach Anspruch 1, **dadurch gekennzeichnet, dass** die Zylinderampulle (3) eine Keilnut (37) hat, die angrenzend an die röhrenförmige Kantenfläche (322) angeordnet ist und die sich in der axialen Richtung erstreckt, wobei das Sitzelement (22) einen Keil (224) hat, der sich von dem ergriffenen Abschnitt (222) nach hinten erstreckt und der dafür angeordnet ist, in und längs der Keilnut (37) zu gleiten.

22. Einwegspritze mit einer eingebauten Zylinderampulle nach Anspruch 1, ferner **gekennzeichnet durch** ein röhrenförmiges Berührungselement (16), das eine röhrenförmige Berührungswand einschließt, welche die Achse umgibt, und das ein hinteres Eingriffsende (162), das verschiebbar in Eingriff mit dem vorderen Wandabschnitt (124) gebracht ist, und ein vorderes Berührungsende (161) einschließt, das kraft einer Gleitbewegung des hinteren Eingriffsendes (162) bewegt werden kann, zwischen einer vorderen Stellung, wobei das Berührungsende (161) vor dem Spitzenende (232) der Nadelkanüle (23) angeordnet ist, um die Haut eines Patienten zu berühren, bevor die Haut des Patienten mit dem Spitzenende (232) durchstochen wird, und einer hinteren Stellung, wobei das Berührungsende (161) eingezogen ist, um das Spitzenende (232) für einen Durchstechvorgang freizulegen.

23. Einwegspritze mit einer eingebauten Zylinderampulle nach Anspruch 22, ferner **gekennzeichnet durch** eine Feder (17), die dafür angeordnet ist, das Berührungsende (161) zu der vorderen Stellung vorzuspannen.

24. Einwegspritze mit einer eingebauten Zylinderampulle nach Anspruch 22, **dadurch gekennzeichnet, dass** der Zylinder (1) eine röhrenförmige vordere Wand (129) hat, die sich von dem hinteren Wandabschnitt (123) in der axialen Richtung nach vom erstreckt und die mit Abstand in radialen Richtungen entfernt von dem vorderen Wandabschnitt (124) angeordnet ist, um einen Gleitkanal (120) zwischen denselben zu definieren, wobei der Gleitkanal (120) dafür konfiguriert ist, das Berührungselement (16) aufzunehmen und die Bewegung des hinteren Eingriffselements (162) im Verhältnis dazu zu führen, wobei die Spritze ferner einen Nadelschützer (25) umfasst, der dafür konfiguriert ist, die röhrenförmige vordere Wand (129) abnehmbar abzudecken, um so die Nadelkanüle (23) zu schützen.

25. Einwegspritze mit einer eingebauten Zylinderampulle nach Anspruch 1, ferner **gekennzeichnet durch** einen Druckkolbenschützer (5), der lösbar auf den hinteren Wandabschnitt (123) geschoben wird, um den Druckkolben (4) zu schützen, und der einen lösbar eingreifenden Bereich (53) hat, der in reibschlüssigen Eingriff mit dem Betätigungsende (42) des Druckkolbens (4) gebracht wird, wenn der Druckkolbenschützer (5) auf den hinteren Wandabschnitt (123) geschoben wird.

## Revendications

1. Seringue jetable comportant une ampoule intégrée, comprenant:
un cylindre (1), englobant des extrémités avant et arrière de cylindre (122, 121), opposées l'une à l'autre le long d'un axe dans une direction axiale, une paroi de cylindre environnante (12), interconnectant lesdites extrémités avant et arrière du cylindre (122, 121) et englobant des parties de paroi avant et arrière (124, 123) agencées respectivement près desdites extrémités avant et arrière du cylindre (122, 121) et définissant respectivement des compartiments avant et arrière (11a, 11b) ;
une canule d'aiguille (23), comportant une extrémité de pointe (232) agencée vers l'avant de ladite extrémité avant du cylindre (122) dans une position d'utilisation, et une extrémité de communication (231) opposée à ladite extrémité de pointe (232) le long de l'axe ;
un élément de siège (22), comportant une partie environnante à saisie (222) entourant l'axe ; et
un élément de saisie (21) retenue de manière amovible sur ladite partie de paroi arrière (123), et configuré de sorte à être engagé par frottement dans ladite partie à saisie (222) pour établir une force de résistance, empêchant un déplacement de ladite partie à saisie (222) par rapport à ladite partie de paroi arrière (123) dans la direction axiale au cours d'une action de percement de ladite canule d'aiguille (23), en vue d'une injection, et permettant le dégagement dudit élément de saisie (21) de ladite partie à saisie (22) pour permettre un déplacement ultérieur dudit élément de siège (22) lorsque ledit élément de saisie (21) est soumis à une force de pression, **caractérisée par** :
une ampoule (3), comportant une paroi tubulaire (32) configurée de sorte à être ajustée dans ledit compartiment arrière (11b), et s'étendant vers ledit élément de saisie (21) pour se terminer au niveau d'une surface de bordure tubulaire (322), et des éléments de joint avant et arrière (35 ; 34 ; 325, 34) espacés l'un de l'autre dans la direction axiale, et formant, en coopération avec ladite paroi tubulaire (32), une chambre à fermeture étanche (31) pour contenir un médicament liquide, ledit élément d'étanchéité arrière (34) étant agencé de sorte à pouvoir se déplacer pour buter contre ledit élément d'étanchéité avant (35, 325) lors d'une course initiale, de sorte à expulser le médicament liquide hors de ladite chambre à fermeture étanche (3 1), plaçant ainsi ladite ampoule (3) dans une position d'épuisement, ledit élément d'étanchéité avant (35, 325) comportant une surface d'accouplement (354, 326) configurée de sorte à permettre la retenue de ladite extrémité de communication (231) par ladite surface d'accouplement et sa traversée de celle-ci, de sorte que ladite canule d'aiguille (23) est en communication de fluide avec ladite chambre à fermeture étanche (31), et que, lors de la butée dudit élément d'étanchéité arrière (34) contre ledit élément d'étanchéité avant (35, 325), dans la position d'épuisement, la force de pression est transmise à travers ladite paroi tubulaire (32) pour pousser ladite surface de bordure tubulaire (322) contre ledit élément de saisie (21) lors d'une course ultérieure, pour entraîner le dégagement dudit élément de saisie (21) de ladite partie à saisie (222), plaçant ainsi ledit élément de siège (22) dans une position dégagée, dans laquelle ladite surface d'accouplement (354, 326) est plus proche de ladite extrémité avant du cylindre (122) que dans la position d'épuisement ;
un piston (4), comportant une extrémité de poussée (432) agencée de sorte à s'engager dans ledit élément d'étanchéité arrière (34) et à déplacer celui-ci vers l'avant le long de ladite paroi tubulaire (32), pour transférer ladite ampoule (3) vers la position d'épuisement, et pour déplacer ladite surface d'accouplement (354) vers l'avant à partir de la position d'épuisement, pour permettre le positionnement dudit élément de siège (22) dans la position dégagée, et une extrémité opérationnelle (42) opposée à ladite extrémité de poussée (432) dans la direction axiale, et agencée de sorte à s'étendre vers l'extérieur de ladite extrémité arrière du cylindre (121) afin d'être soumise à une force externe, plus élevée lors de la course ultérieure que lors de la course initiale ; et
un élément poussoir (24), agencé dans ledit compartiment avant (11a), de sorte qu'après le positionnement dudit élément de siège (22) dans la position dégagée, ledit élément poussoir (24) pousse ledit élément de siège (22) ensemble avec ladite ampoule (3) vers ladite extrémité arrière du cylindre (121) pour rétracter ladite extrémité de pointe (232) de ladite canule d'aiguille (23) dans ledit cylindre (1), positionnant ainsi ladite canule d'aiguille (23) dans une position d'évacuation.

2. Seringue jetable comportant une ampoule intégrée selon la revendication 1, **caractérisée en ce que** ledit piston (4) englobe :
un premier corps de piston (43) comportant ladite extrémité de poussée (432) agencée de sorte à s'engager dans ledit élément d'étanchéité arrière (34) et à se déplacer avec celui-ci, et une première partie d'extrémité arrière (433) opposée à ladite extrémité de poussée (432) ; et
un deuxième corps de piston (41), englobant ladite extrémité opérationnelle (42) et une deuxième partie d'extrémité avant (413), opposée à ladite extrémité opérationnelle (42), et retenue de manière amovible avec ladite première partie d'extrémité arrière (433) par une force de frottement supérieure à la force externe lors de la course initiale, pour permettre le déplacement dudit élément d'étanchéité arrière (34) vers l'avant par la force externe, le long de ladite paroi tubulaire (32), pour positionner ainsi ladite ampoule (3) dans la position d'épuisement, et inférieure à la force externe lors de la course ultérieure, pour permettre le déplacement dudit deuxième corps de piston (41) par rapport audit premier corps de piston (43), de sorte à transférer la force externe vers ladite paroi tubulaire (32), pour pousser ainsi ladite surface de bordure tubulaire (322) contre ledit élément de saisie (21) par la force de pression.

3. Seringue jetable comportant une ampoule intégrée selon la revendication 2, **caractérisée en ce que** ladite paroi tubulaire (32) de ladite ampoule (3) comporte une extrémité de paroi arrière (321) opposée à ladite surface de bordure tubulaire (322), et un épaulement annulaire arrière (33, 36), s'étendant radialement et vers l'intérieur à partir de ladite extrémité arrière de la paroi (321), ledit deuxième corps du piston (41) comportant une butée (421) agencée sur ladite deuxième partie d'extrémité avant (413) dudit deuxième corps de piston (41), et configurée de sorte à buter contre ledit épaulement annulaire arrière (33, 36) lorsque ladite ampoule (3) se trouve dans la position d'épuisement, pour transférer la force externe vers ladite paroi tubulaire (32).

4. Seringue jetable comportant une ampoule intégrée selon la revendication 3, **caractérisée en ce que** ledit épaulement annulaire arrière (33, 36) est configuré de sorte à empêcher un déplacement vers l'arrière dudit élément d'étanchéité arrière (34) par rapport à ladite paroi tubulaire (32).

5. Seringue jetable comportant une ampoule intégrée selon la revendication 2, **caractérisée en ce que** ladite paroi du cylindre (12) comporte une partie de retenue (126) adjacente à ladite extrémité arrière du cylindre (121), ledit deuxième corps de piston (41) étant configuré de sorte que ladite extrémité opérationnelle (42) est retenue par ladite partie de retenue (126) après le positionnement dudit élément de siège (22) dans la position dégagée, pour empêcher un déplacement vers l'arrière dudit deuxième corps de piston (41).

6. Seringue jetable comportant une ampoule intégrée selon la revendication 2, **caractérisée en ce que** ladite première partie d'extrémité arrière (433) et ladite deuxième partie d'extrémité avant (413) comportent respectivement une rainure de retenue annulaire (431) et une saillie de retenue annulaire (411), mutuellement accouplées et engagées l'une dans l'autre pour produire la force de frottement.

7. Seringue jetable comportant une ampoule intégrée selon la revendication 1, **caractérisée en ce que** ledit élément de siège (22) est agencé de sorte à fixer ladite canule d'aiguille (23) et à permettre une extension vers l'arrière de ladite extrémité de communication (231) de ladite canule d'aiguille (23), le traversant, ladite extrémité de communication (231) étant configurée de sorte à être pointue, afin de percer ladite surface d'accouplement (354) dudit élément d'étanchéité avant (35), pour établir ainsi une communication entre ladite canule d'aiguille (23à) et ladite chambre à fermeture étanche (31).

8. Seringue jetable comportant une ampoule intégrée selon la revendication 7, **caractérisée en ce que** ladite surface d'accouplement (354) dudit élément d'étanchéité avant (35) comporte une cavité (351) s'étendant vers l'intérieur et en direction de ladite chambre à fermeture étanche (31), de sorte à recevoir ladite partie à saisie (222) dudit élément de siège (22) lorsque ledit élément de siège (22) se trouve dans la position dégagée, assurant ainsi le maintien de la liaison entre ladite ampoule et l'élément de siège (22) lorsque ledit élément de siège (22) est poussé vers ladite extrémité arrière du cylindre (121).

9. Seringue jetable comportant une ampoule intégrée selon la revendication 7, **caractérisée en ce que** ledit élément d'étanchéité avant (35) comporte une bride annulaire (353), agencée vers l'avant de ladite paroi tubulaire (32) et entraînée à buter contre ladite surface de bordure tubulaire (322) pour assurer un engagement étanche au fluide entre ledit élément d'étanchéité avant (35) et ladite paroi tubulaire (32).

10. Seringue jetable comportant une ampoule intégrée selon la revendication 7, **caractérisée en ce que** ladite partie de paroi arrière (123) englobe un premier segment de paroi (123a), formé d'une seule pièce avec ladite partie de paroi avant (124) et s'étendant vers l'arrière de celle-ci pour recevoir ledit élément de siège (22) et ledit élément de saisie (21), et un deuxième segment de paroi (123b) accouplé de manière amovible audit premier segment de paroi (123a) pour recevoir ladite ampoule (3) et ledit piston (4).

11. Seringue jetable comportant une ampoule intégrée selon la revendication 10, **caractérisée en outre par** un premier capuchon d'extrémité (6), connecté audit premier segment de paroi (123a) pour protéger ladite extrémité de communication (231) de ladite canule d'aiguille (23), et un deuxième capuchon d'extrémité (7), connecté audit deuxième segment de paroi (123b) pour protéger ladite ampoule (3).

12. Seringue jetable comportant une ampoule intégrée selon la revendication 1, **caractérisée en ce que** ladite ampoule (3) comporte une partie de retenue tubulaire de l'aiguille (327), s'étendant vers l'avant à partir de ladite surface d'accouplement (326) le long de l'axe et retenant ladite extrémité de communication (231) de ladite canule d'aiguille (23) pour établir une communication entre ladite canule d'aiguille (23) et la chambre à fermeture étanche (31).

13. Seringue jetable comportant une ampoule intégrée selon la revendication 12, **caractérisée en outre par** un protecteur de la pointe (25), emmanché de manière amovible sur ladite paroi du cylindre (12), pour protéger ladite canule d'aiguille (23), et une garniture d'étanchéité élastomère (26) incorporée dans ledit protecteur de la pointe (25), de sorte que lorsque ledit protecteur de la pointe est emmanché sur ladite paroi du cylindre (12), ladite extrémité de pointe (232) de ladite canule d'aiguille (23) est bloquée dans ladite garniture d'étanchéité élastomère (26).

14. Seringue jetable comportant une ampoule intégrée selon la revendication 1, **caractérisée en ce que** ledit compartiment arrière (11b) a un diamètre plus grand que ledit compartiment avant (11a), de sorte à former un épaulement annulaire avant (125) entre eux, s'étendant vers l'axe et se terminant au niveau d'un bord périphérique interne (141) entourant ladite canule d'aiguille (23).

15. Seringue jetable comportant une ampoule intégrée selon la revendication 14, **caractérisée en ce que** ledit élément de siège (22) englobe une surface de butée avant environnante (223), agencée de sorte à faire face audit compartiment avant (11a), et une surface d'extrémité de siège arrière (225) opposée à ladite surface de butée avant environnante (223) et faisant face à ladite surface d'accouplement (354) pour retenir ladite extrémité de communication (231) dans un état stationnaire, pour faciliter le passage de ladite extrémité de communication (231) à travers ladite surface d'accouplement (354).

16. Seringue jetable comportant une ampoule intégrée selon la revendication 15, **caractérisée en ce que** ledit élément de siège (22) englobe un support tubulaire (226) s'étendant de ladite surface de butée avant environnante (223) dans ledit compartiment avant (11a) et espacé dudit bord périphérique interne (141) dans des directions radiales par un dégagement inséré recevant une extrémité inférieure (242) dudit élément de poussée (24), pour permettre la butée dudit élément poussoir (24) contre ladite surface de butée avant environnante (223).

17. Seringue jetable comportant une ampoule intégrée selon la revendication 16, **caractérisée en ce que** ledit support tubulaire (226) s'étend à travers ledit compartiment avant (11a) pour s'engager dans ladite extrémité avant du cylindre (122).

18. Seringue jetable comportant une ampoule intégrée selon la revendication 17, **caractérisée en ce que** ladite surface de butée avant environnante (223) est agencée de sorte à être espacée dudit épaulement annulaire avant (125), pour exposer ainsi ladite extrémité inférieure (242) dudit élément poussoir (24).

19. Seringue jetable comportant une ampoule intégrée selon la revendication 18, **caractérisée en ce que** ledit cylindre (1) comporte un élément d'espacement tubulaire (18), agencé entre ledit épaulement annulaire avant (125) et ladite surface de butée avant environnante (223) dudit élément de siège (22), et renfermant ladite extrémité inférieure exposée (242) dudit élément poussoir (24), ledit élément d'espacement tubulaire (18) étant configuré de sorte à être formé d'une seule pièce avec ledit épaulement annulaire avant (125).

20. Seringue jetable comportant une ampoule intégrée selon la revendication 16, **caractérisée en ce que** ledit élément de siège (22) englobe une surface étagée annulaire (227) espacée dudit épaulement annulaire avant (125) dans la direction axiale, et s'étendant radialement et vers l'extérieur pour rejoindre ladite partie à saisie environnante (222), et plusieurs éléments d'espacement à ailettes (221) déplacés angulairement l'un par rapport à l'autre autour de l'axe, chacun étant agencé entre ladite surface annulaire étagée (227) et ledit épaulement annulaire avant (125), de sorte à caler ladite surface annulaire étagée (227) contre ledit épaulement annulaire avant (125).

21. Seringue jetable comportant une ampoule intégrée selon la revendication 1, **caractérisée en ce que** ladite ampoule (3) comporte une rainure de clavette (37) agencée près de ladite surface de bordure tubulaire (322) et s'étendant dans ladite direction axiale, ledit élément de siège (22) comportant une clavette (224) s'étendant vers l'arrière à partir de ladite partie à saisie (222) et agencée de sorte à glisser dans ladite rainure de clavette (37) et le long de celle-ci.

22. Seringue jetable comportant une ampoule intégrée selon la revendication 1, **caractérisée en outre par** un élément de contact tubulaire (16), englobant une paroi de contact tubulaire entourant l'axe, et comportant une extrémité d'engagement arrière (162) engagée de manière coulissante dans ladite partie de paroi avant (124), et une extrémité de contact avant (161) pouvant être déplacée par l'intermédiaire d'un mouvement de glissement de ladite extrémité d'engagement arrière (162) entre une position avant, dans laquelle ladite extrémité de contact (161) est agencée vers l'avant de ladite extrémité de pointe (232) de ladite canule d'aiguille (23), pour contacter la peau d'un patient avant de percer la peau du patient par ladite extrémité de pointe (232), et une position arrière, dans laquelle ladite extrémité de contact (161) est rétractée pour exposer ladite extrémité de pointe (232) en vue d'une action de percement.

23. Seringue jetable comportant une ampoule intégrée selon la revendication 22, **caractérisée en outre par** un ressort (17) agencé de sorte à pousser ladite extrémité de contact (161) vers la position avant.

24. Seringue jetable comportant une ampoule intégrée selon la revendication 22, **caractérisée en ce que** ledit cylindre (1) comporte une paroi tubulaire avant (129), s'étendant vers l'avant à partir de ladite partie de paroi arrière (123), dans la direction axiale, et espacée de ladite partie de paroi avant (124) dans des directions radiales pour définir un canal de glissement (120) entre elles, ledit canal de glissement (120) étant configuré de sorte à recevoir ledit élément de contact (16) et à guider le déplacement de ladite extrémité d'engagement arrière (162) par rapport à celui-ci, ladite seringue comprenant en outre un protecteur d'aiguille (25), configuré de sorte à recouvrir de manière amovible ladite paroi tubulaire avant (129), pour protéger ladite canule d'aiguille (23).

25. Seringue jetable comportant une ampoule intégrée selon la revendication 1, **caractérisée en outre par** un protecteur du piston (5), emmanché de manière amovible sur ladite partie de paroi arrière (123) pour protéger ledit piston (4), et comportant une région d'engagement amovible (53) engagée par frottement dans ladite extrémité opérationnelle (42) dudit piston (4) lorsque ledit protecteur du piston (5) est emmanché sur ladite partie de paroi arrière (123).
